# EUROPÄISCHE PATENTSCHRIFT

(11) **EP 0 797 574 B1**
(45) Veröffentlichungstag und Bekanntmachung des Hinweises auf die Patenterteilung: **17.03.2004**
(21) Anmeldenummer: 95941658.7
(22) Anmeldetag: 04.12.1995
(51) Int. Cl.: C07D 273/04, C07D 413/04, A01N 43/88

(54) **1,3,4-OXADIAZIN-DERIVATE MIT PESTIZIDER WIRKUNG**
1,3,4-OXADIAZINE DERIVATIVES HAVING A PESTICIDE EFFECT
DERIVES D'1,3,4 OXADIAZINE AYANT UN EFFET PESTICIDE

(30) Priorität: 16.12.1994 DE 4444865
(43) Veröffentlichungstag der Anmeldung: 01.10.1997
(73) Patentinhaber: Bayer CropScience AG, 40789 Monheim (DE)
(72) Erfinder: KLEEFELD, Gerd, D-41468 Neuss (DE); KANELLAKOPULOS, Johannes, D-41542 Dormagen (DE); WACHENDORFF-NEUMANN, Ulrike, D-56566 Neuwied (DE)
(86) Internationale Anmeldenummer: PCT/EP1995/004760
(87) Internationale Veröffentlichungsnummer: WO 1996/018624

(56) Entgegenhaltungen:
- WO-A-93/22311
- WO-A-95/18116
- US-A- 4 782 066
- PESTIC. BIOCHEM. PHYSIOL. (PCBPBS,00483575);93; VOL.47 (1); PP.1-7, UNIV. WATERLOO;DEP. BIOL.; WATERLOO; N2L 3G1; ON; CAN. (CA), ISMAIL S M M ET AL 'Effect of dihydrooxadiazines on the octopamine-sensitive adenylate cyclase complex of two-spotted spider mite, Tetranychus urticae Koch' in der Anmeldung erwähnt
- CHEMICAL ABSTRACTS, vol. 83, no. 1, 7.Juli 1975 Columbus, Ohio, US; abstract no. 10171j, POTHEKIN '5,6-Dihydro-4H-1,3,4-oxadiazines'

## Beschreibung

Die Erfindung betrifft neue 1,3,4-Oxadiazin-Derivate, mehrere Verfahren zu ihrer Herstellung und ihre Verwendung zur Bekämpfung von tierischen Schädlingen.

Es ist bereits bekannt, daß bestimmte 1,3,4-Oxadiazin-Derivate, wie z.B. das 2-(4-Bromphenyl)-4-(2-fluoreth-1-yl)-4H-5,6-dihydro-1,3,4-oxadiazin akarizide Eigenschaften aufweisen (vgl. Pesticide Biochemistry and Physiology 47, 1-7 (1993)).

Die Wirkungshöhe und/oder Wirkungsdauer dieser vorbekannten Verbindungen ist jedoch, insbesondere gegen bestimmte Organismen oder bei niedrigen Anwendungskonzentrationen nicht in allen Anwendungsgebieten völlig zufriedenstellend.

Es wurden neue 1,3,4-Oxadiazin-Derivate der Formel (I) gefunden, 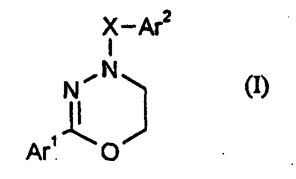 in welcher
- Ar¹: steht für Phenyl, das gegebenenfalls einfach bis dreifach, gleich oder verschieden substituiert ist durch Fluor, Chlor, Brom, C₁-C₄-Alkyl, C₁-C₄-Alkoxy, C₁-C₄-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃, SCHF₂, Nitro oder Cyano oder für Thienyl.
- Ar²: steht für Phenyl, das gegebenenfalls einfach bis vierfach, gleich oder verschieden substituiert ist durch Fluor, Chlor, Brom, C₁-C₁₈-Alkyl, C₁-C₆-Alkoxy-C₁-C₈-alkyl, einfach bis sechsfach, gleich oder verschieden durch Fluor oder Chlor substituiertes C₁-C₈-Alkoxy,
einfach bis fünffach, gleich oder verschieden durch' Fluor oder Chlor substituiertes C₁-C₂-Alkyl,
C₁-C₁₈-Alkoxy, -(OC₂H₄)₁₋₃-O-C₁-C₆-alkyl, C₁-C₁₂-Alkylthio, einfach bis sechsfach, gleich oder verschieden durch Fluor oder Chlor substituiertes C₁-C₈-Alkylthio,
jeweils gegebenenfalls durch C₁-C₄-Alkyl, C₁-C₄-Alkoxy, Cyclohexyl oder Phenyl substituiertes Cyclohexyl oder Cyclohexyloxy,
gegebenenfalls einfach oder zweifach, gleich öder verschieden durch Fluor, Chlor oder CF₃ substituiertes Pyridyloxy,
jeweils gegebenenfalls einfach bis dreifach, gleich oder verschieden durch C₁-C₁₂-Alkyl, Fluor, Chlor, Brom, Cyano, CF₃, C₁-C₄-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxyethylenoxy, C₁-C₄-Alkylthio, einfach bis sechsfach, gleich oder verschieden durch Fluor oder Chlor substituiertes C₁-C₄-Alkylthio oder Trimethylsilyl substituiertes Phenyl, Benzyl, Phenethyl, Phenoxy, Phenoxymethyl, Phenylthio, Phenethyloxy, Benzyloxy, Benzylthio oder Styryl.
- X: steht für die Gruppierung -(CHR¹)ₘ-(CHR²)ₙ-, worin
R¹ für Wasserstoff steht,
R² für Wasserstoff steht,
m für 0 oder 1 steht und
n für 0 oder 1 steht.

Weiterhin wurde gefunden, daß man die neuen 1,3,4-Oxadiazin-Derivate der Formel (I) erhält, wenn man
a) Säurehydrazide der Formel (II)

   Ar¹-CO-NH-NH-X-Ar² (II)

   in welcher
   - Ar¹, Ar² und X: die oben angegebene Bedeutung haben,
   mit Ethan-Derivaten der Formel (III)

   Y¹-CH₂-CH₂-Y² (III)

   in welcher
   - Y¹ und Y²: gleich oder verschieden sind und jeweils für eine Abgangsgruppe stehen,
   in Gegenwart einer Base und in Gegenwart eines Verdünnungsmittels umsetzt;
   oder
b) Säurehydrazide der Formel (IV) 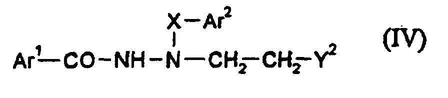 in welcher
   - Ar¹, Ar², X und Y²: die oben angegebene Bedeutung haben,
   gegebenenfalls in Gegenwart eines Katalysators, gegebenenfalls in Gegenwart einer Base und gegebenenfalls in Gegenwart eines Verdünnungsmittels cyclisiert;
   oder
c) Säurehydrazide der Formel (V) 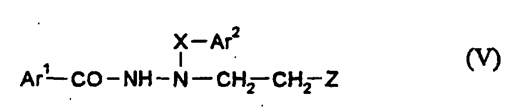 in welcher
   - Ar¹, Ar² und X: die oben angegebene Bedeutung haben und
   - Z: für -OH oder -O-COR steht, wobei
   R für Alkyl steht,
   in Gegenwart einer Säure und gegebenenfalls in Gegenwart eines Verdünnungsmittels cyclisiert;
   oder
d) 1,3,4-Oxadiazini-Derivate der Formel (VI) 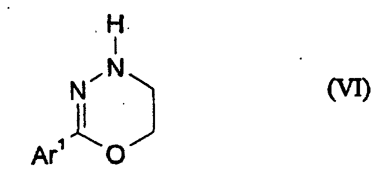 in welcher
   - Ar¹: die oben angegebene Bedeutung hat,
   mit Verbindungen der Formel (VII)

   Y¹-(CHR¹)ₘ-(CHR²)ₙ-Ar² (VII)

   in welcher
   - Ar², R¹, R², Y¹, m und n: die oben angegebene Bedeutung haben, und mindestens einer der Indizes m oder n für 1 steht,
   in Gegenwart einer Base und gegebenenfalls in Gegenwart eines Verdünnungsmittels umsetzt;
   oder
e) 1,3,4-Oxadiazin-Derivate der Formel (Ia) 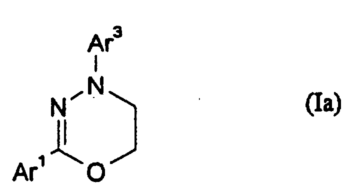 in welcher
   - Ar¹: die oben angegebene Bedeutung hat und
   - Ar³: für Halogenaryl oder Halogenhetaryl steht
   mit Boronsäuren der Formel (VIII)

   Ar⁴-B(OH)₂ (VIII)

   in welcher
   - Ar⁴: für gegebenenfalls substituiertes Aryl steht
   in Gegenwart einer Base und in Gegenwart eines Verdünnungsmittels umsetzt.

Weiterhin wurde gefunden, daß die neuen 1,3,4-Oxadiazin-Derivate der Formel (I) sehr gut zur Bekämpfung von tierischen Schädlingen geeignet sind. Sie zeichnen sich insbesondere durch hohe Wirksamkeit gegen Arthropoden aus.

Überraschenderweise zeigen die erfindungsgemäßen 1,3,4-Oxadiazin-Derivate der Formel (I) eine erheblich bessere Wirksamkeit gegenüber tierischen Schädlingen als die konstitutionell ähnlichsten vorbekannten Verbindungen, wie beispielsweise das 2-(4-Bromphenyl)-4-(2-fluoreth-1-yl)-4H-5,6-dihydro-1,3,4-oxadiazin.

Die erfindungsgemäßen Verbindungen sind durch die Formel (I) allgemein definiert.

Bevorzugte Substituenten bzw. Bereiche der in den oben und nachstehend erwähnten Formeln aufgeführte Reste werden im folgenden erläutert.
- Ar¹: steht ganz besonders hervorgehoben für Phenyl, das einfach bis dreifach, gleich oder verschieden substituiert ist durch Fluor, Chlor, Trifluormethyl oder Trifluormethoxy oder für Thienyl.
- Ar²: steht ganz besonders hervorgehoben für Phenyl, das gegebenenfalls einfach bis dreifach, gleich oder verschieden durch Fluor, Chlor, Brom, C₁-C₆-Alkyl oder durch jeweils gegebenenfalls einfach oder zweifach, gleich oder verschieden durch Fluor, Chlor, Trifluormethyl oder Trifluormethoxy substituiertes Phenyl, Phenoxy oder Benzyloxy substituiert ist.
- X: steht ganz besonders hervorgehoben für die Gruppierung -(CH₂)ₘ-, worin
m für 0 oder 1 steht.

Die oben bei der Definition der erfindungsgemäßen Verbindungen genannten Kohlen wasserstoffreste, wie Alkyl, sind - auch in Verbindung mit Heteroatomen, wie Alkoxy - soweit möglich, jeweils geradkettig oder verzweigt.

Beispiele für die Substituenten Ar¹ und Ar² sind in den folgenden Tabellen 1 und 2 aufgeführt:

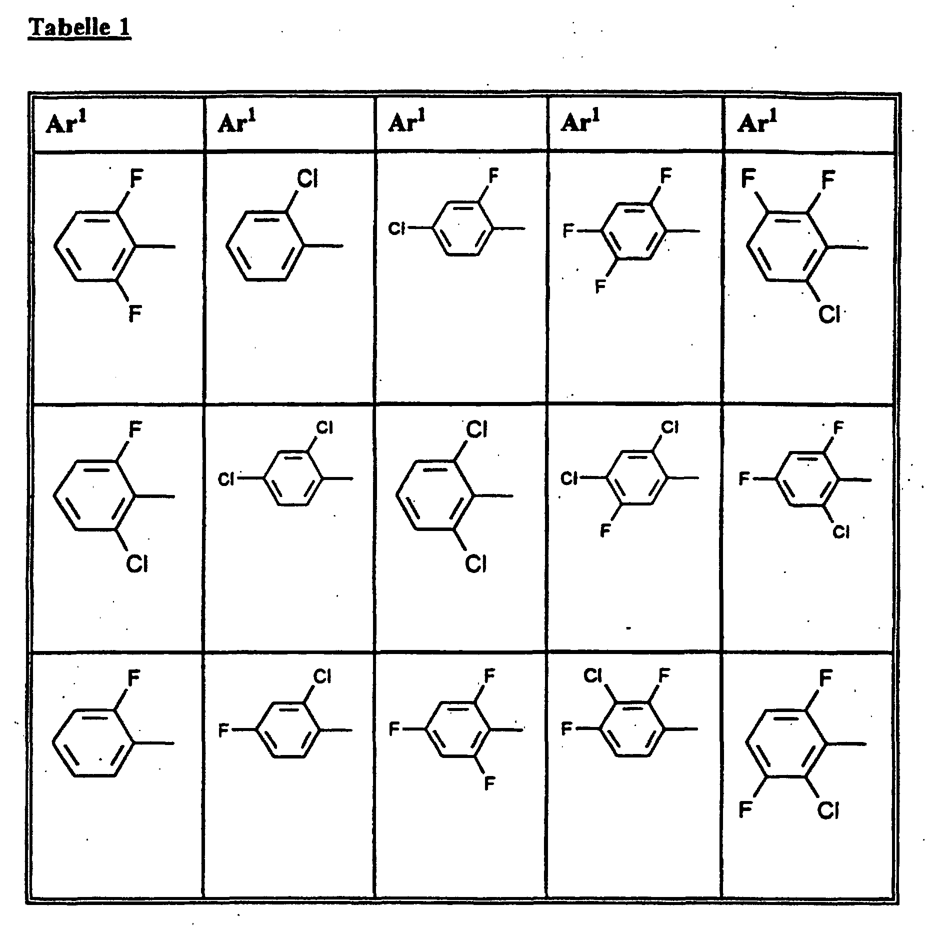

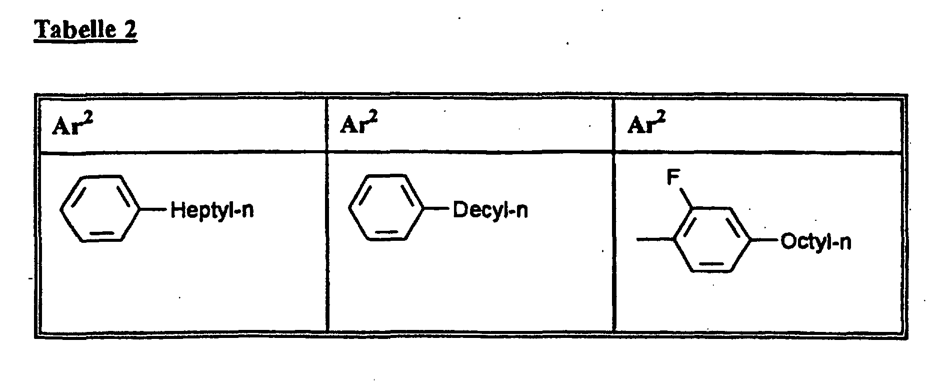

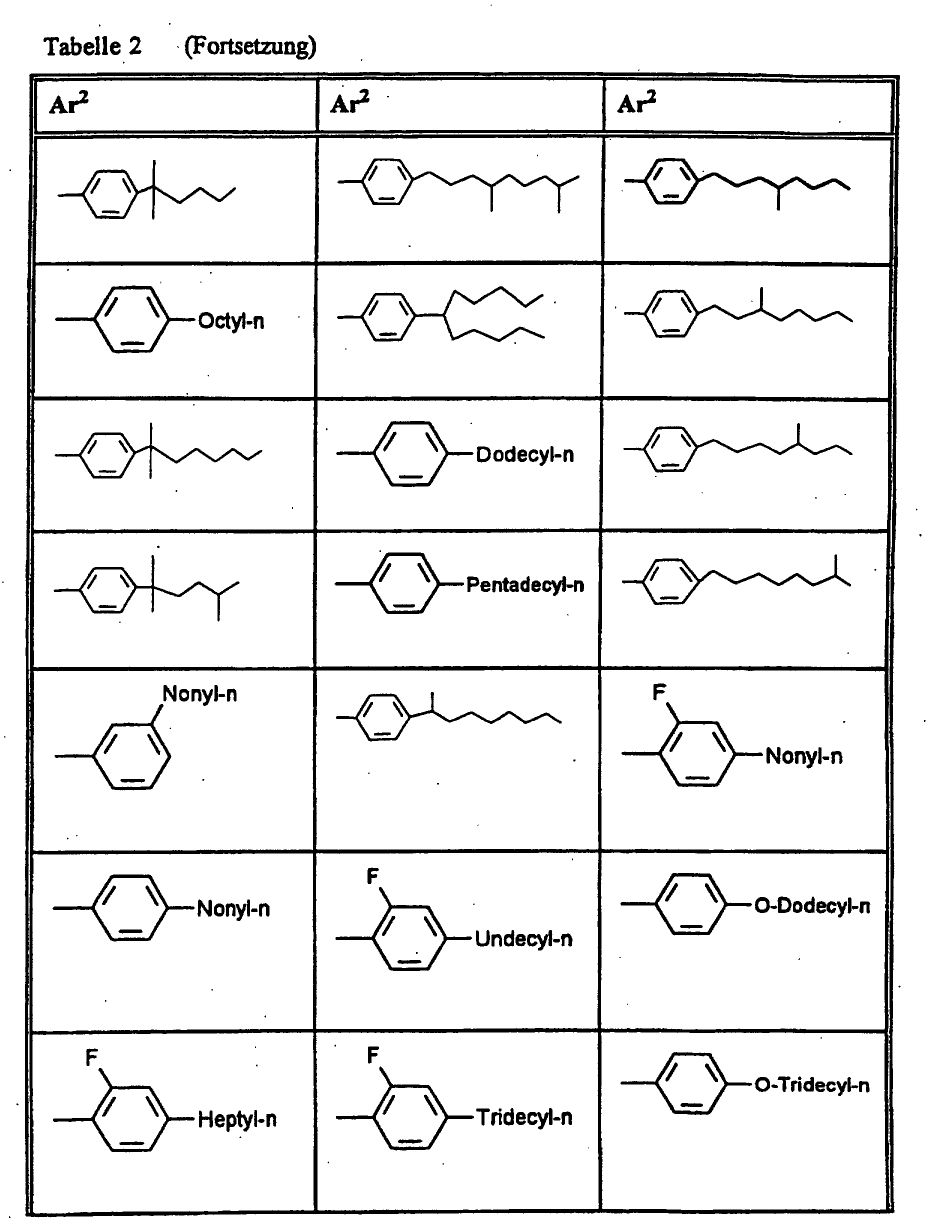

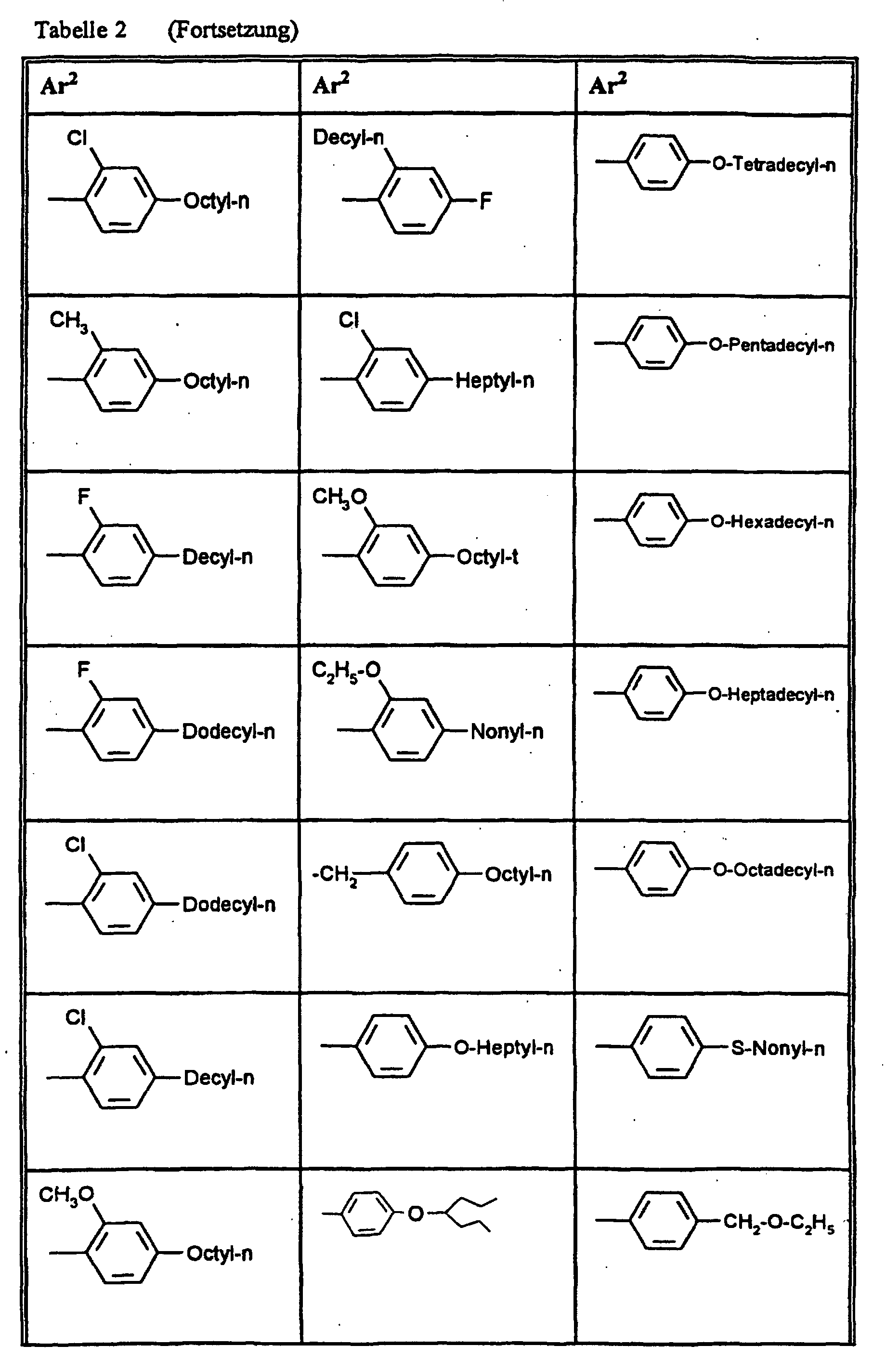

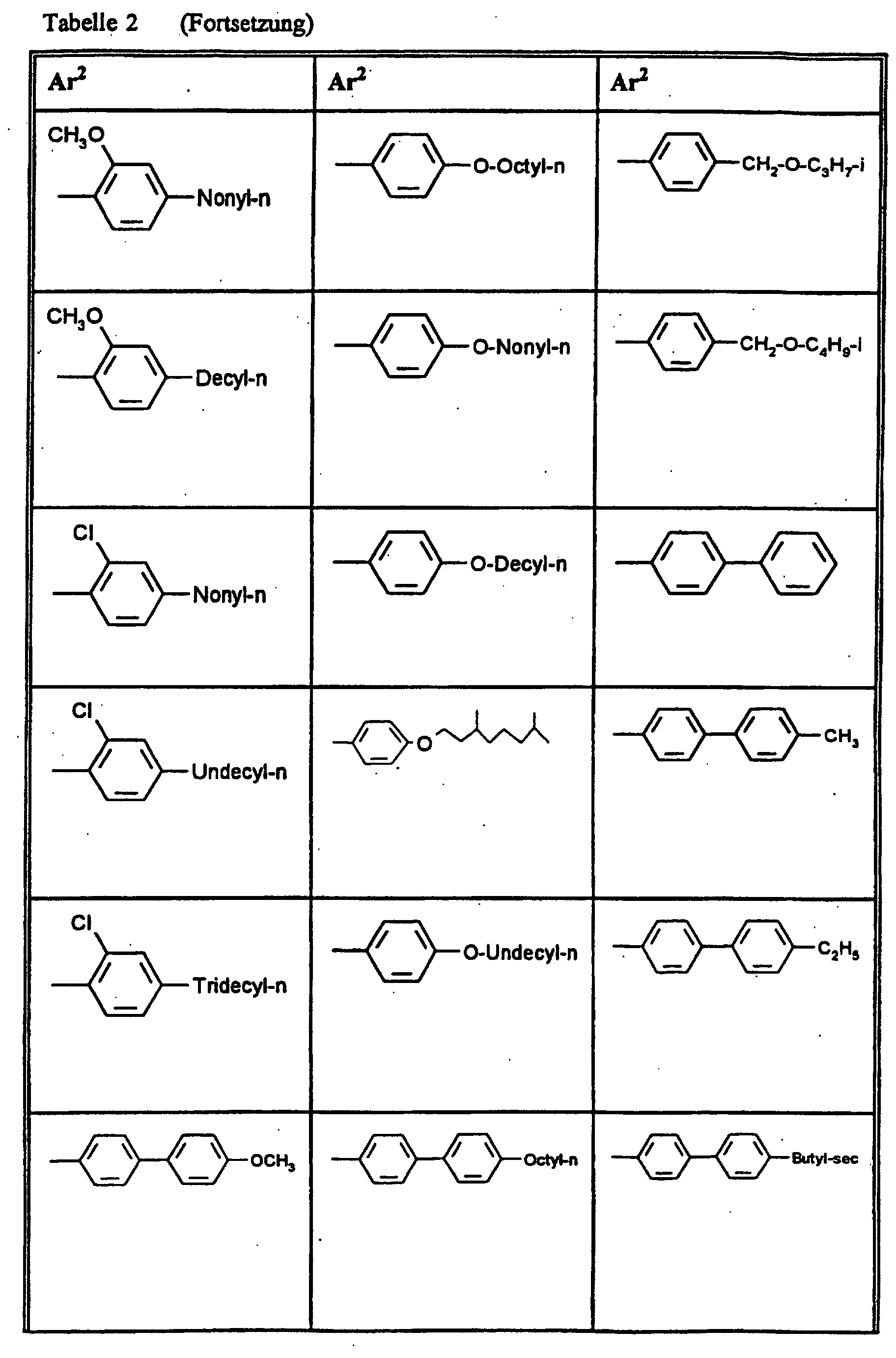

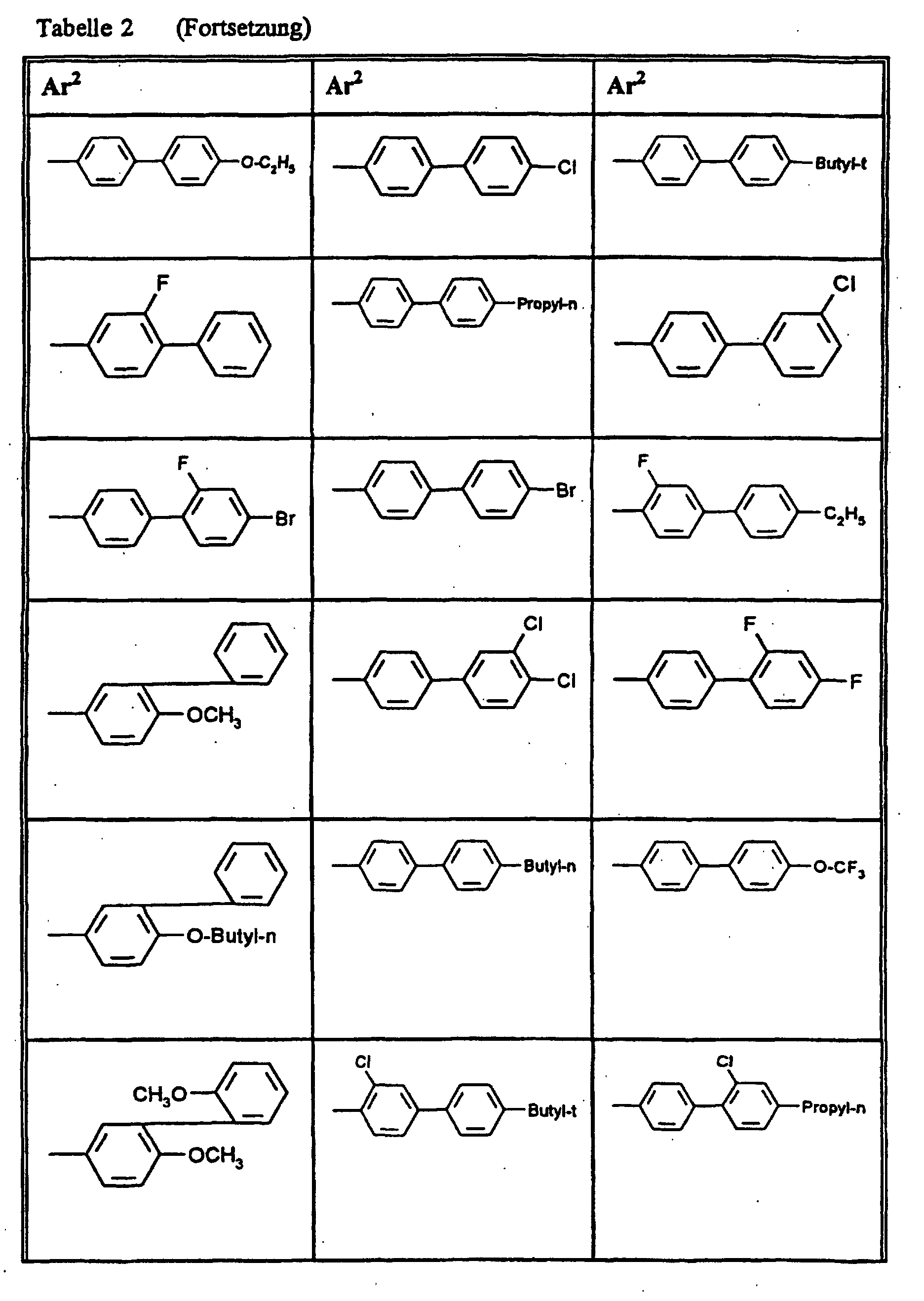

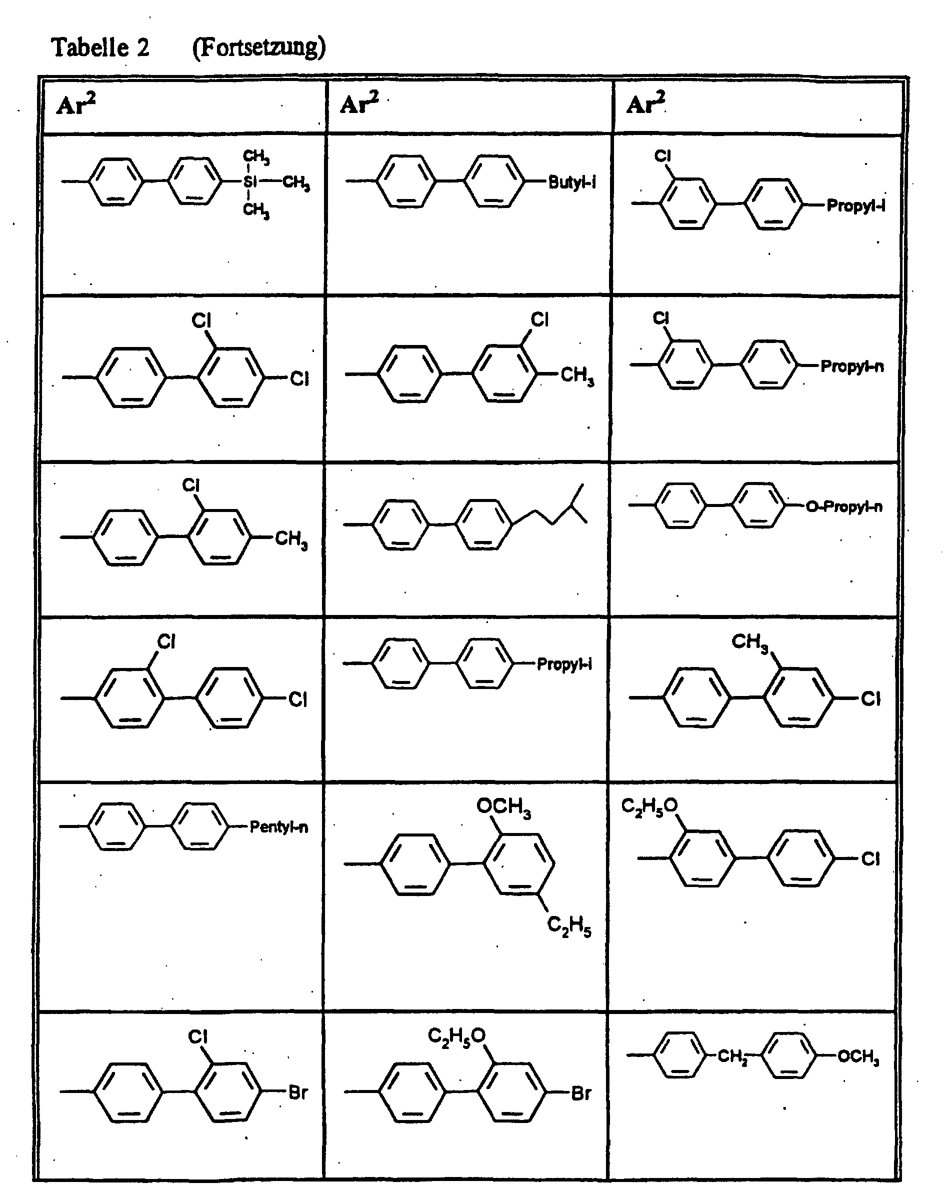

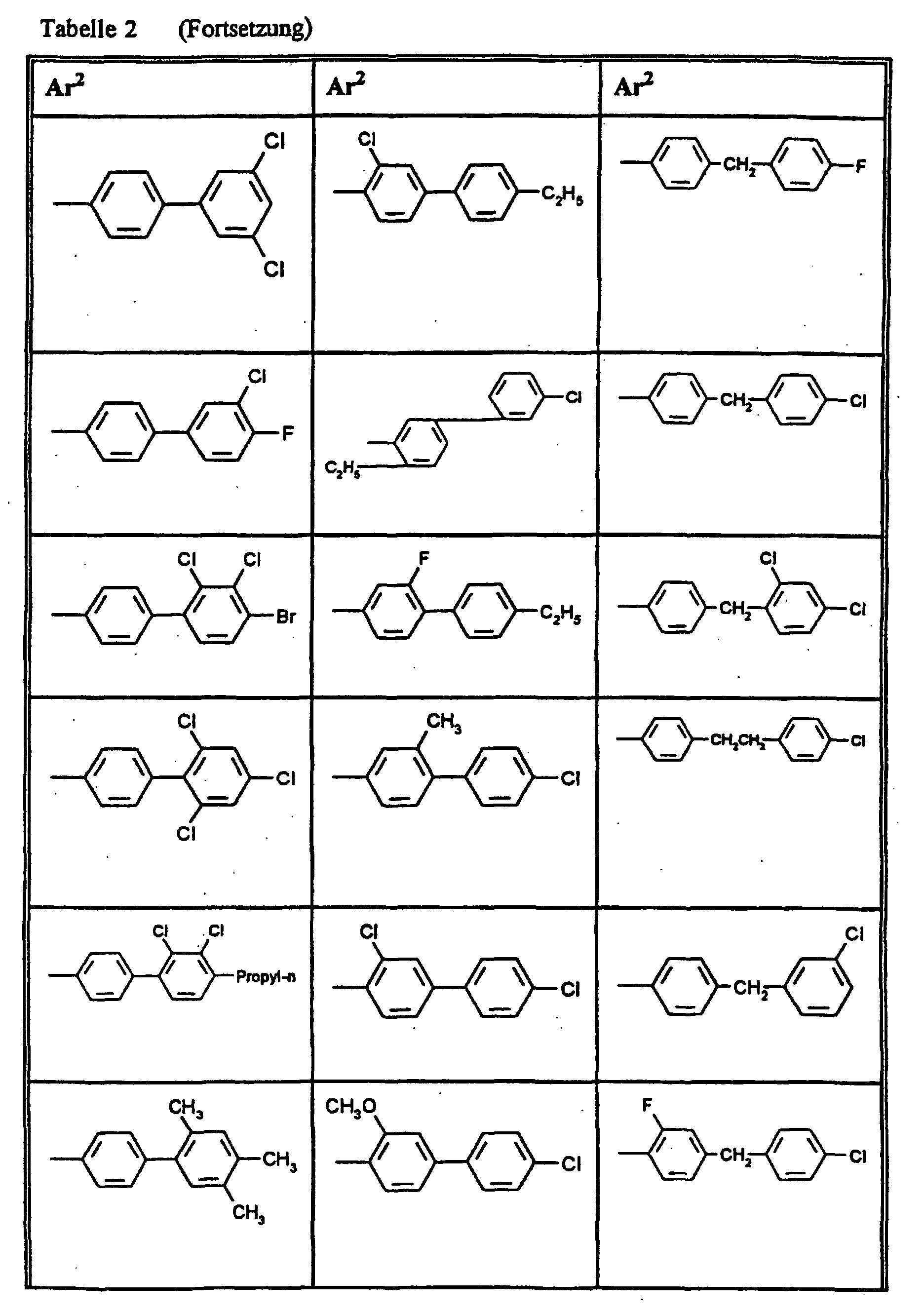

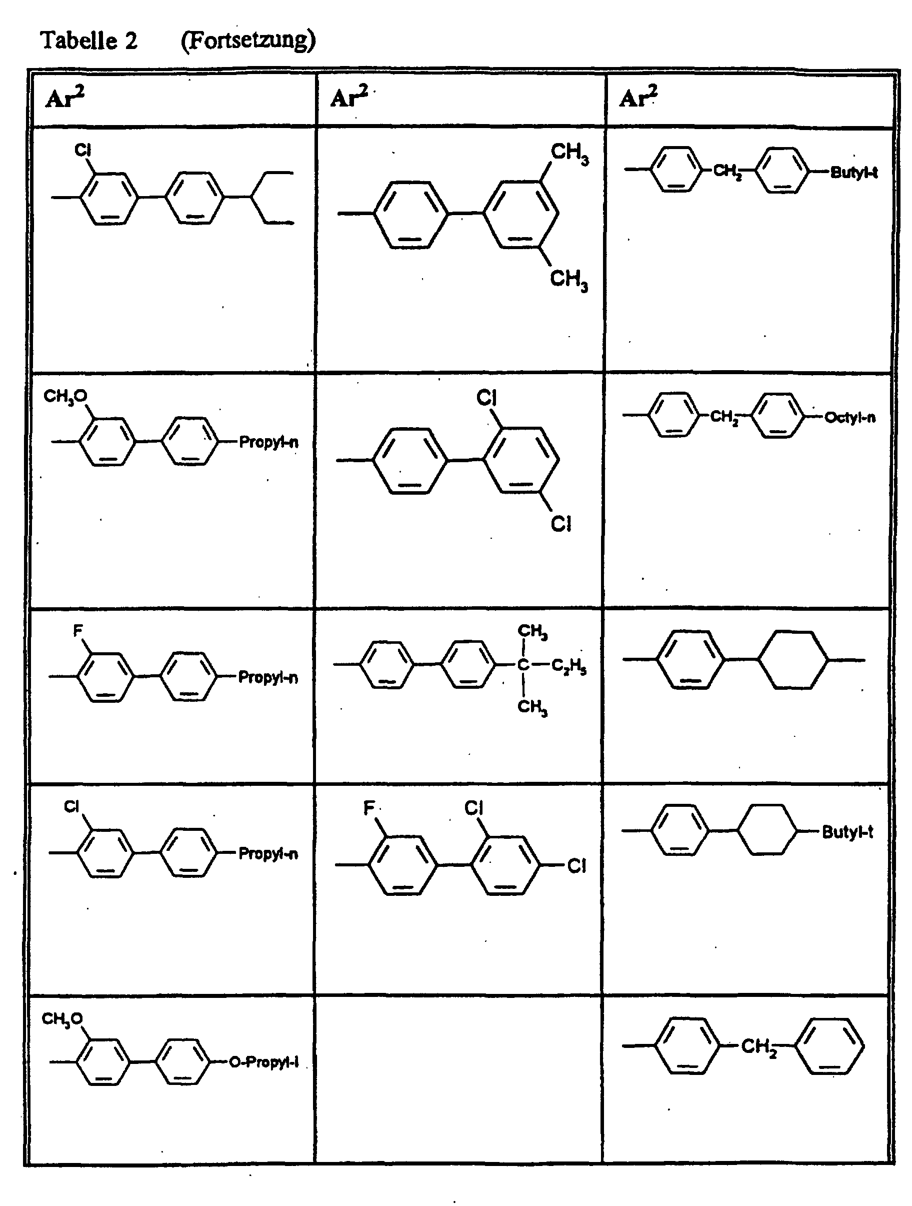

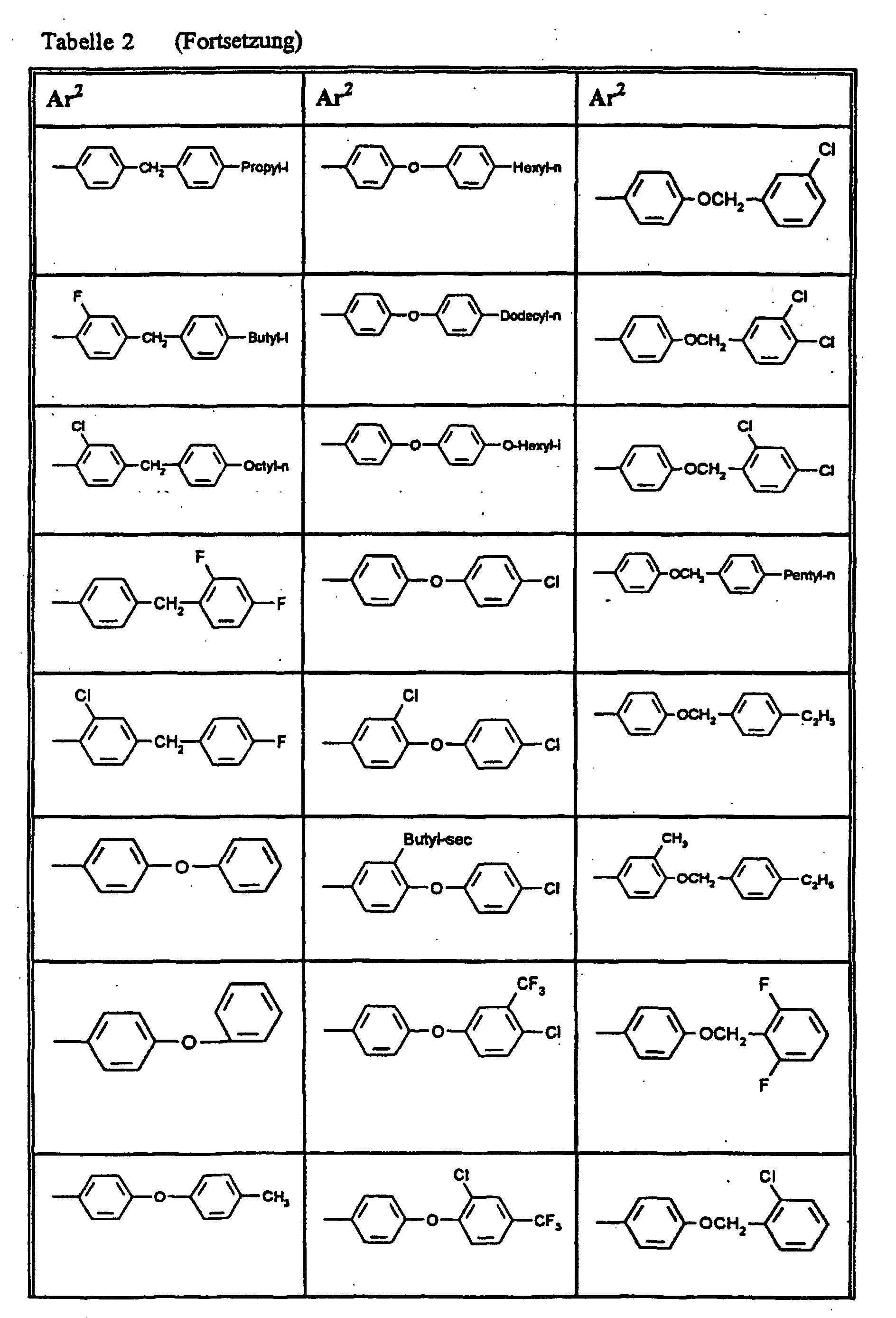

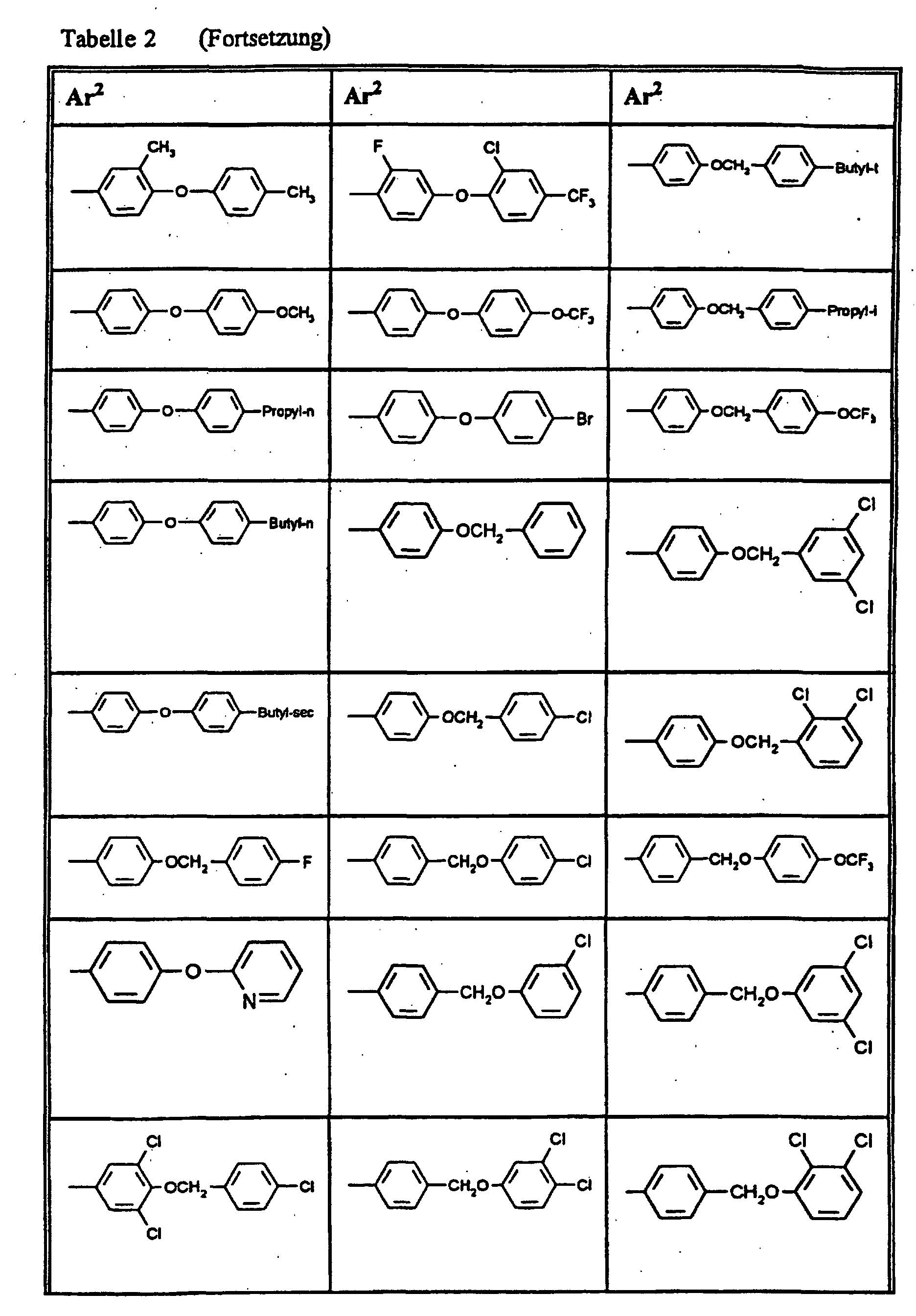

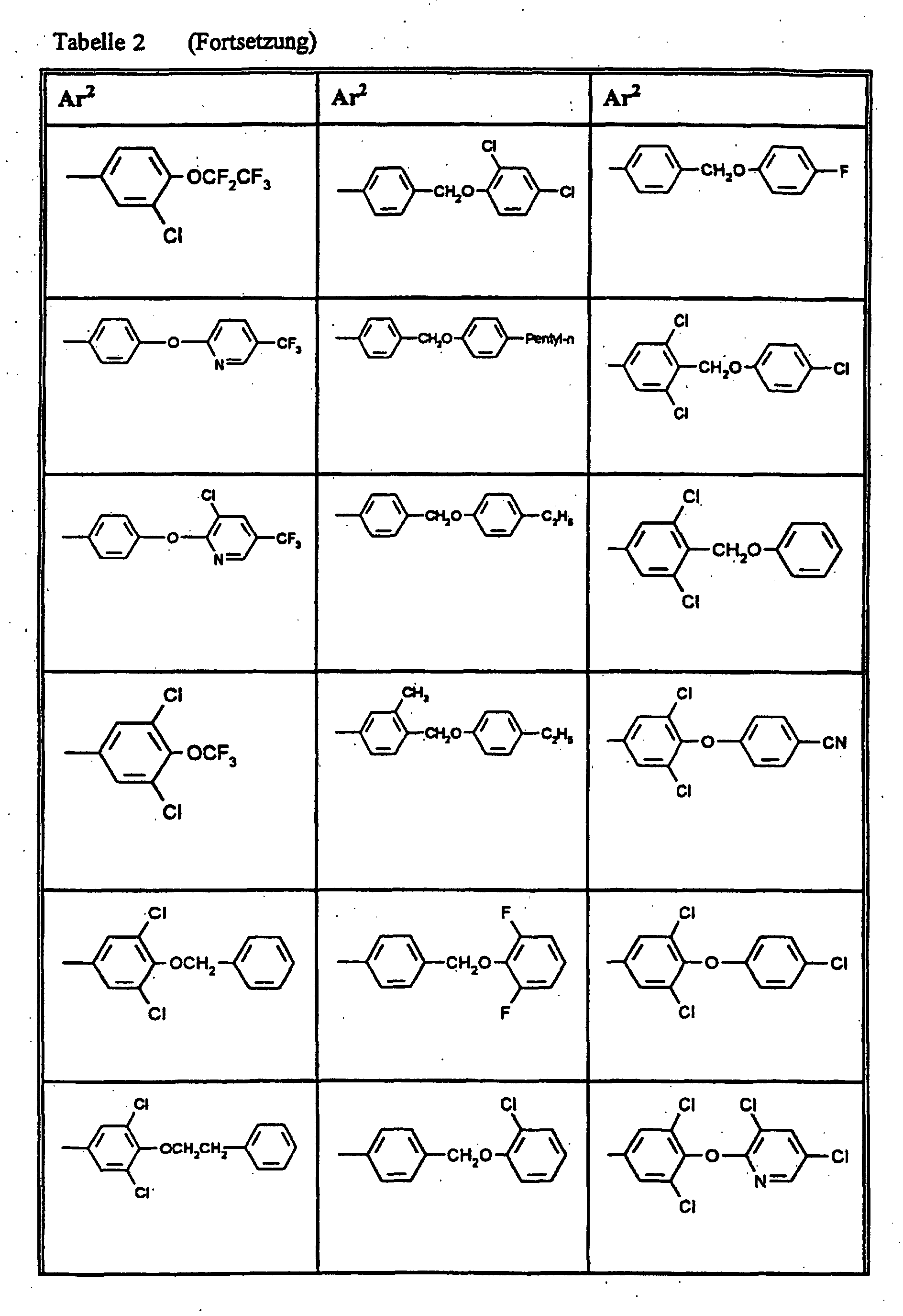

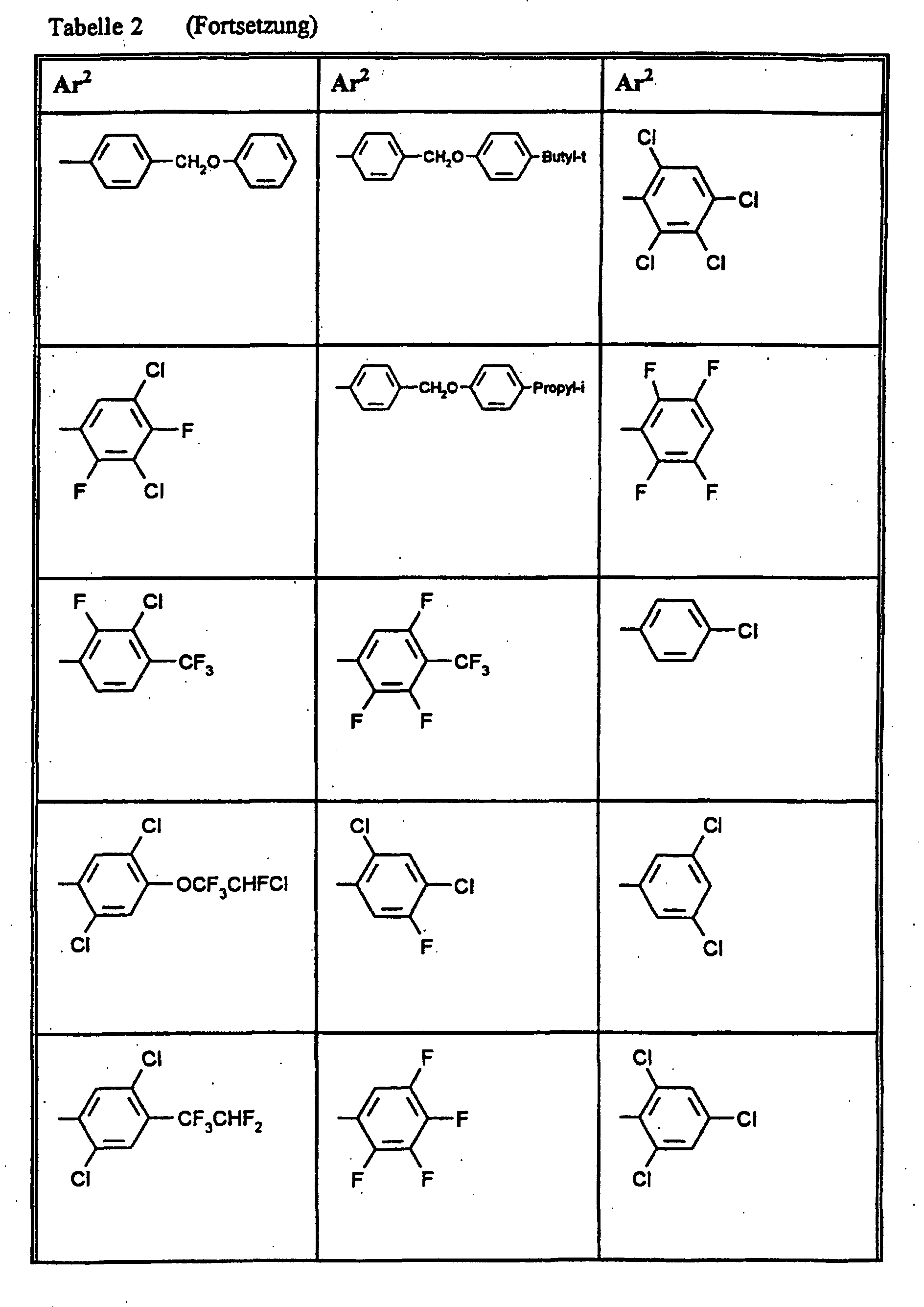

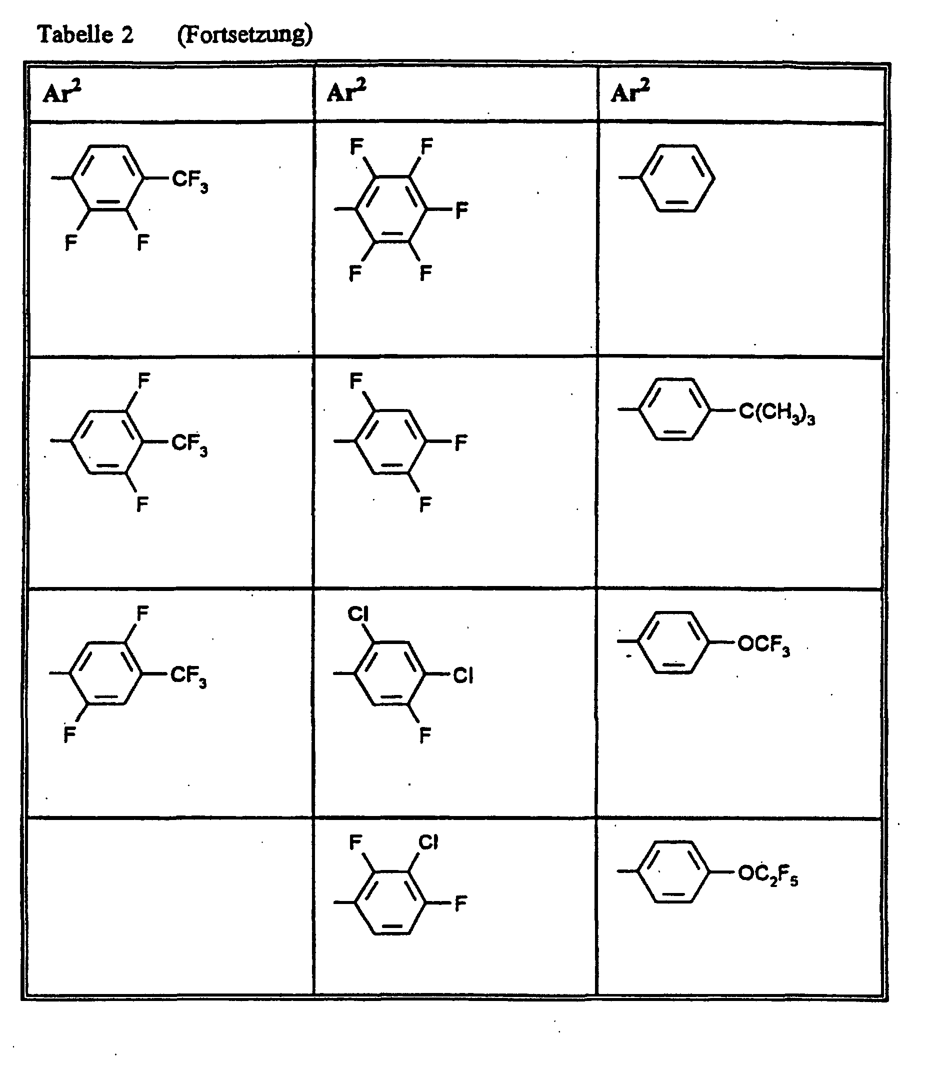

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in' welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß ganz besonders hervorgehoben sind die Verbindungen der Formel (I), in welchen eine Kombination der vorstehend als ganz besonders hervorgehoben aufgeführten Bedeutungen vorliegt.

Verwendet man gemäß Verfahren (a) z.B. 1-(2,6-Difluorbenzoyl)-2-(4-bromphenyl)-hydrazin und 1-Brom-2-fluorethan als Ausgangsstoffe, so kann der Verlauf des erfindungsgemäßen Verfahrens durch das folgende Reaktionsschema wiedergegeben werden:

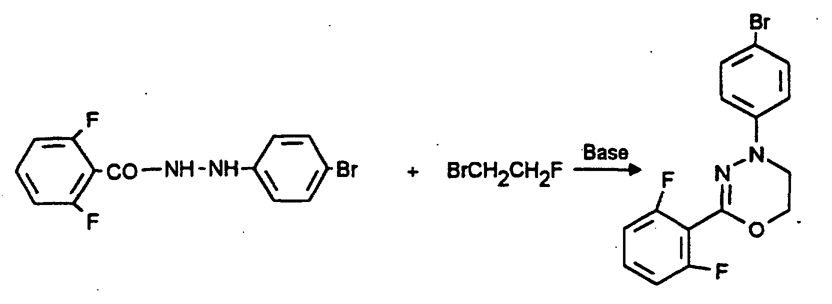

Verwendet man gemäß Verfahren (b) z.B. 1-(2,6-Difluorbenzoyl)-2-(2,4,6-trichlorphenyl)-2-(2-brometh-1-yl)-hydrazin als Ausgangsstoff und gegebenenfalls Kaliumjodid als Katalysator, so kann der Verlauf des erfindungsgemäßen Verfahrens durch das folgende Reaktionsschema wiedergegeben werden:

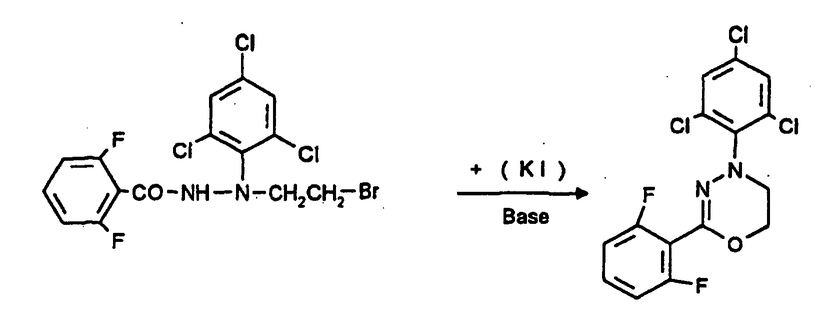

Verwendet man gemäß Verfahren (c) z.B. 1-(2,6-Difluorbenzoyl)-2-(2,4,6-trichlorphenyl)-2-(2-hydroxyeth-1-yl)-hydrazin als Ausgangsstoff, so kann der Verlauf des erfindungsgemäßen Verfahrens durch das folgende Reaktionsschema wiedergegeben werden:

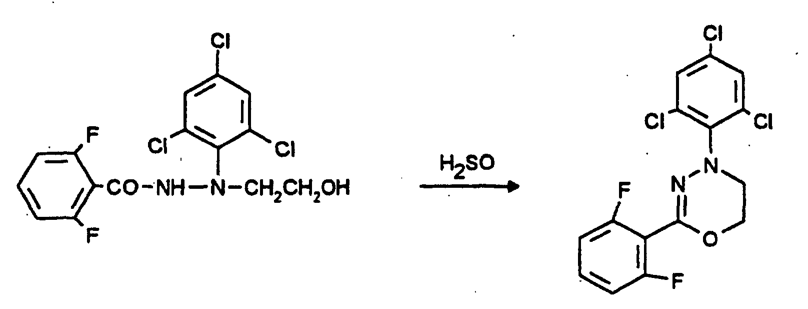

Verwendet man gemäß Verfahren (d) z.B. 1-(2,6-Difluorphenyl)-4H-5,6-dihydro-1,3,4-oxadiazin und Benzylbromid als Ausgangsstoffe, so kann der Verlauf des erfindungsgemäßen Verfahrens durch das folgende Reaktionsschema wiedergegeben werden:

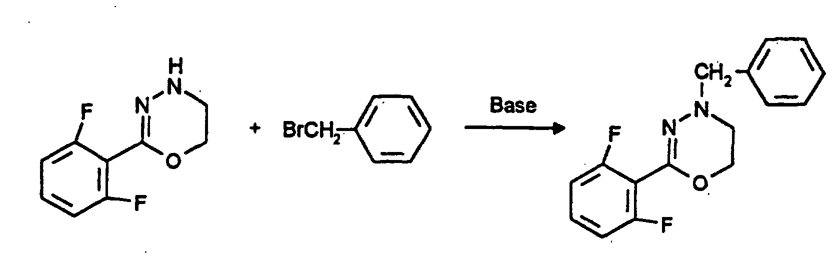

Verwendet man gemäß Verfahren (e) z.B. 2-(2,6-Difluorphenyl)-4-(4-bromphenyl-4H-5,6-dihydro-1,3,4-oxadiazin und 4-Trifluormethoxyphenylboronsäure als Ausgangsstoffe, so kann der Verlauf des erfindungsgemäßen Verfahrens durch das folgende Reaktionsschema wiedergegeben werden:

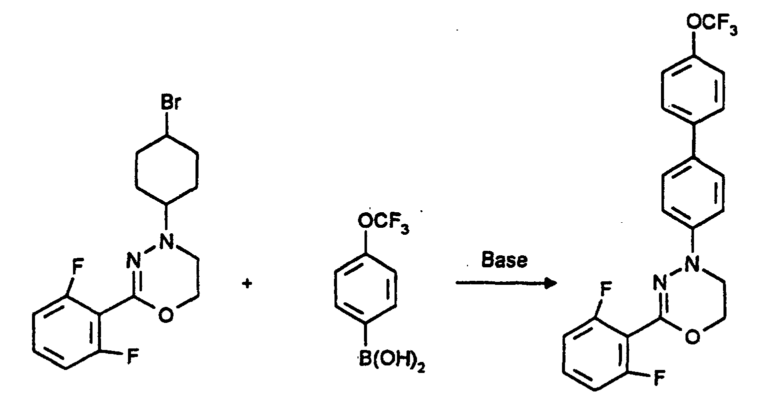

Die zur Durchführung des erfindungsgemäßen Verfahrens (a) als Ausgangsstoffe benötigten Säurehydrazide sind durch die Formel (II) allgemein definiert.

Die Säurehydrazide der Formel (II) sind bekannt bzw. können in allgemein bekannter Art und Weise erhalten werden (vgl. hierzu z.B. US 2 167 793; Revue Roumaine de Chimie 29 (2), 219-22 (1984); Liebigs Annalen der Chemie 1975, 1264). Man erhält Säurehydrazide der Formel (II) beispielsweise, wenn man Säurehydrazide der Formel (IX)

Ar¹-CO-NH-NH₂ (IX)

in welcher
- Ar¹: die oben angegebene Bedeutung hat,
mit Aldehyden der Formel (X)

Ar²-X-CHO (X)

in welcher
- Ar² und X: die oben angegebene Bedeutung haben, wobei jedoch mindestens einer der Indizes m oder n für 0 steht,
in Gegenwart eines Verdünnungsmittels, z.B. aromatischen Kohlenwasserstoffen oder Alkoholen wie beispielsweise Toluol oder Ethanol und gegebenenfalls in Gegenwart eines Kondensationsmittels, z.B. einer Säure wie beispielsweise p-Toluolsulfonsäure bei Temperaturen zwischen 20 und 120°C umsetzt (vgl. auch Farmaco Ed. Sci. 39 (1984), 414 sowie die Herstellungsbeispiele); und die dabei entstehenden Acylidenderivate der Formel (XI)

Ar¹-CO-NH-N=CH-X-Ar² (XI)

in welcher
- Ar¹, Ar² und X: die oben angegebene Bedeutung haben
reduziert, zum Beispiel mittels Wasserstoff in Gegenwart eines Katalysators, (beispielsweise Palladium/Kohle) oder mittels Borhydriden (beispielsweise Natrium- oder Calciumborhydrid), gegebenenfalls in Gegenwart eines Verdünnungsmittels, beispielsweise Alkoholen wie Methanol oder Ethanol bei Temperaturen zwischen 0 und 100°C sowie gegebenenfalls unter erhöhtem Druck (vgl. auch Liebigs Annalen der Chemie 1975, 1264; Farmaco Ed. Sci. 16 (1961) 832 sowie die Herstellungsbeispiele);
oder wenn man aktive Carbonsäurederivate, beispielsweise der Formeln (XIIa-c)

Ar¹-CO-Cl (XIIa)

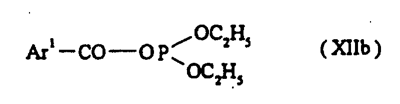

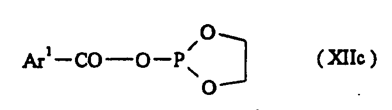

in welchen
- Ar¹: die oben angegebene Bedeutung hat
mit Hydrazinen der Formel (XIII)

Ar² -X-NH-NH₂ (XIII)

in welcher
- Ar² und X: die oben angegebene Bedeutung haben,
in Gegenwart eines Verdünnungsmittels, z.B. gegebenenfalls halogenierten aliphatischen oder aromatischen Kohlenwasserstoffenwie beispielsweise Methylenchlorid oder Toluol und gegebenenfalls in Gegenwart einer Base, z.B. organischen Stickstoffbasen, wie beispielsweise Triethylamin bei Temperaturen zwischen 0 und 100°C umsetzt (vgl. auch US 2 617 793; Revue Roumaine de Chimie 29 (2), 219-22 (1984)).

Die Säurehydrazide der Formel (IX), die Aldehyde der Formel (X), die Carbonsäurederivate der Formeln (XIIa-c) sowie die Hydrazine der Formel (XIII) sind bekannt (vgl. z.B. die o.g. Literaturangaben) bzw. können in allgemein bekannter Art und Weise erhalten werden.

Die außerdem zur Durchführung des erfindungsgemäßen Verfahrens (a) als Ausgangsstoffe benötigten Ethan-Derivate sind durch die Formel (III) allgemein definiert. In der Formel (III) sind Y¹ und Y² gleich oder verschieden und stehen für allgemein übliche Abgangsgruppen, wie Halogen, insbesondere Fluor, Chlor oder Brom, Alkylsulfonyloxy, insbesondere Methylsulfonyloxy, oder gegebenenfalls substituiertes Arylsulfonyloxy, insbesondere Phenylsulfonyloxy, p-Chlorphenylsulfonyloxy oder Tolylsulfonyloxy.

Die Ethan-Derivate der Formel (III) sind allgemein bekannte Verbindungen der organischen Chemie.

Die zur Durchführung des erfindungsgemäßen Verfahrens (b) als Ausgangsstoffe benötigten Säurehydrazide sind durch die Formel (IV) allgemein definiert. Y² steht für eine der Abgangsgruppen, die bereits oben im Zusammenhang mit der Beschreibung der Ethan-Derivate der Formel (III) genannt wurden.

Die Säurehydrazide der Formel (IV) treten bei der Durchführung des erfindungsgemäßen Verfahrens (a) als Zwischenstufe auf und können hierbei isoliert werden. Sie können auch erhalten werden, indem in allgemein bekannter Weise die Abgangsgruppe Z in den Säurehydraziden der Formel (V) in die Abgangsgruppe Y² überführt wird.

Die zur Durchführung des erfindungsgemäßen Verfahrens (c) als Ausgangsstoffe benötigten Säurehydrazide sind durch die Formel (V) allgemein definiert. Z steht vorzugsweise für eine der Abgangsgruppen Hydroxy oder -O-CO-R, wobei R bevorzugt für C₁-C₄-Alkyl steht, insbesondere für Methyl.

Die Säurehydrazide der Formel (V) werden beispielsweise erhalten, indem man Säurehydrazide der Formel (II) gemäß den Bedingungen des erfindungsgemäßen Verfahrens (a) mit bekannten Ethan-Derivaten der Formel (IIIa)

Y¹-CH₂-CH₂-Z (IIIa)

in welcher
- Y¹ und Z: die oben angegebene Bedeutung haben,
umsetzt.

Die zur Durchführung des erfindungsgemäßen Verfahrens (d) als Ausgangsstoffe benötigten 1,3,4-Oxadiazin-Derivate sind durch die Formel (VI) allgemein definiert.

Die 1,3,4-Oxadiazin-Derivate der Formel (VI) sind bekannt bzw. (vgl. z.B. Pesticide Science 1993, 38, 309-314; Pesticide Biochemistry and Physiology 47, 1-7 (1993)) bzw. können nach den dort beschriebenen Verfahren erhalten werden.

Die außerdem zur Durchführung des erfindungsgemäßen Verfahrens (d) als Ausgangsstoffe benötigten Verbindungen sind durch die Formel (VII) allgemein definiert.

Die Verbindungen der Formel (VII) sind allgemein bekannte Verbindungen der organischen Chemie.

Die zur Durchführung des erfindungsgemäßen Verfahrens (e) als Ausgangsstoffe benötigten 1-,3,4-Oxadiazin-Derivate sind durch die Formel (Ia) allgemein definiert.

Ar³ steht vorzugsweise für Jod- oder Bromphenyl, besonders bevorzugt für 4-Iod- oder 4-Bromphenyl.

Die 1,3,4-Oxadiazin-Derivate der Formel (Ia) sind erfindungsgemäße Verbindungen und gemäß den erfindungsgemäßen Verfahren (a) bis (d) erhältlich.

Die außerdem zur Durchführung des erfindungsgemäßen Verfahrens (e) als Ausgangsstoffe benötigten Boronsäuren sind durch die Formel (VIII) allgemein definiert. In der Formel (VIII) steht Ar⁴ bevorzugt für gegebenenfalls einfach bis dreifach, gleich oder verschieden durch C₁-C₁₂-Alkyl, Halogen, Cyano, C₁-C₄-Halogenalkyl, C₁-C₆-Alkoxy, C₁-C₆-Halogenalkoxy, C₁-C₆-Alkoxy-C₁-C₆-alkyl, C₁-C₆-Alkoxy-ethylenoxy, C₁-C₆-Alkylthio, C₁-C₆-Halogenalkylthio oder Trimethylsilyl substituiertes Phenyl, besonders bevorzugt für gegebenenfalls einfach bis dreifach, gleich oder verschieden durch C₁-C₁₂-Alkyl, Fluor, Chlor, Brom, Cyano, CF₃, C₁-C₄-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxyethylenoxy, C₁-C₄-Alkylthio, einfach bis sechsfach, gleich oder verschieden durch Fluor oder Chlor substituiertes C₁-C₄-Alkylthio oder Trimethylsilyl substituiertes Phenyl und besonders bevorzugt für gegebenenfalls durch Trifluormethyl oder Trifluormethoxy substituiertes Phenyl.

Die Boronsäuren der Formel (VIII) sind allgemein bekannte Verbindungen der organischen Chemie.

Als Verdünnungsmittel kommen bei der Durchführung der erfindungsgemäßen Verfahren (a) bis (e) alle unter den jeweils gegebenen Reaktionsbedingungen inerten organischen Lösungsmittel in Frage. Sie können gegebenenfalls in Mischung mit Wasser verwendet werden. Bevorzugt verwendet werden Kohlenwasserstoffe wie Toluol, Xylol, Tetralin, Hexan, Cyclohexan, Halogenkohlenwasserstoffe wie Methylenchlorid, Chloroform, Chlorbenzol, o-Dichlorbenzol, Alkohole wie Methanol, Ethanol, Glykol, die isomeren Propanole, Butanole, Pentanole, Ether wie Diethylether, Diisopropylether, Dimethoxyethan, Tetrahydrofuran, Dioxan, Nitrile wie Acetonitril oder Butyronitril, Amide wie Dimethylformamid, Sulfoxide wie Dimethylsulfid, ferner Sulfolan.

Als Base kommen bei der Durchführung der erfindungsgemäßen Verfahren (a), (b), (d) und (e) alle üblichen Säureakzeptoren in Frage. Vorzugsweise verwendbar sind tertiäre Amine wie Triethylamin, Pyridin, Diazabicyclooctan (DABCO), Diazabicycloundecen (DBU), Diazabicyclononen (DBN), N,N-Dimethylanilin, ferner Erdalkalimetalloxide wie Magnesium- oder Calciumoxid, außerdem Alkaliund Erdalkalimetallcarbonate wie Natriumcarbonat, Kaliumcarbonat oder Calciumcarbonat, Alkalihydroxide wie Natrium- oder Kaliumhydroxid, ferner Alkoholate wie Natriumethanolat oder Kalium-tert.-butylat.

Das erfindungsgemäße Verfahren (b) wird gegebenenfalls in Gegenwart eines Katalysators durchgeführt. Als geeignete Katalysatoren kommen beispielsweise infrage: Kaliumjodid, Pyridin oder DABCO.

Das erfindungsgemäße Verfahren (c) wird in Gegenwart einer Säure durchgeführt. Prinzipiell kommen anorganische oder organische Säuren infrage. Bevorzugt verwendet werden beispielsweise Schwefelsäure, Methansulfonsäure, Benzolsulfonsäure oder p-Toluolsulfonsäure.

Die Reaktionstemperaturen können bei der Durchführung der erfindungsgemäßen Verfahren (a) bis (e) in einem größeren Bereich variiert werden. Im allgemeinen arbeitet man bei Temperaturen zwischen 0°C und 150°C, bevorzugt zwischen 0°C und 100°C beziehungsweise bei der Siedetemperatur des verwendeten Lösungsmittels.

Die erfindungsgemäßen Verfahren (a) bis (e) werden im allgemeinen unter Normaldruck durchgeführt; es ist aber auch möglich gegebenenfalls unter erhöhtem Druck zu arbeiten.

Bei der Durchführung der erfindungsgemäßen Verfahren (a) bis (e) arbeitet man allgemein mit äquimolaren Mengen, wobei gegebenenfalls 0,01 bis 0,1 Mol an Katalysator, 0,5 bis 1,0 Mol an Säure sowie 1 bis 10 Mol an Base eingesetzt werden. Die Aufarbeitung erfolgt nach üblichen Methoden.

Die Wirkstoffe eignen sich zur Bekämpfung von tierischen Schädlingen, vorzugsweise Arthropoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma laaigerum, Hyalopterus arundinis, Macrosiphum avenae, Myzus spp., Pemphigus spp., Phorodon humuli, Phylloxera vastatrix, Rhopalosiphum padi, Empoasca spp., Euscelis bilobatus, Nephotettix cincticeps, Lecahium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys, spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.. Aus der Ordnung der Arachnida z.B. Scorpio maurus, Latrodectus mactans.

Aus der Ordnung der Acarina z.B. Acarus, siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Milben, wie beispielsweise gegen die Bohnenspinnmilbe (Tetranychus urticae) einsetzen.

Die Wirkstoffe können in die üblichen Formulierungen überführt werden, wie Lösungen, Emulsionen, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

### Als feste Trägerstoffe kommen in Frage:

z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emu-Igier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann in seinen andelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazole-5-carboxanilid; 2,6-Dichloro-N-(4-trifluoromethylbenzyl)benzamid; (E)-2-Methoxyimino-N-methyl-2-(2-phenoxyphenyl)-acetamid; 8-Hydroxychinolinsulfat; Methyl-(E)-2-{2-[6-(2-cyanophenoxy)pyrimidin-4-yloxy]-phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate, Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfemaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil, Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin, Bacillus thuringiensis, 4-Bromo-2-(4-chlorphenyl)-1-(ethoxymethyl)-5-(trifluoromethyl)-1H-pyrrole-3-carbanitrile, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chloretoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb, HCH, Heptenophos, Hexaflumuron, Hexythiazox,
Imidacloprid, Iprobenfos, Isazophos, Isofenphos, Isopiocarb, Isoxathion, Ivermectin,
Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, Pirimiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Der erfindungsgemäße Wirkstoff kann ferner in seinen handelsüblichen Formulierungen sowie in den aus deisen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiteren Bereichen variieren. Die Wirkstoffkonzentration der Anwendungsformen kann von 0,0000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die Herstellung und die Verwendung der erfindungsgemäßen Wirkstoffe geht aus den nachfolgenden Beispielen hervor.

### Herstellungsbeispiele

### Beispiel 1

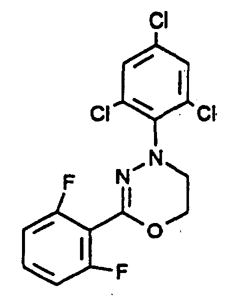

### (Verfahren a)

1,75 g (5,0 mmol) 2-(2,6-Difluorbenzoyl)-1-(2,4,6-trichlorphenyl)-hydrazin werden mit 1,38 g (10,0 mmol) Kaliumcarbonat 20 Minuten bei Raumtemperatur in 4 ml Dimethylacetamid gerührt. Nach Zugabe von 0,94 g (5,0 mmol) Dibromethan wird über Nacht bei 90°C gerührt.
Das Reaktionsgemisch wird auf Essigester/Wasser gegeben, die organische Phase über Natriumsulfat getrocknet und im Vakuum eingeengt.
Der Rückstand wird an Kieselgel 60 (Essigester/Hexan (1:3)) chromatographiert. Man erhält 0,48 g (25% der Theorie) 2-(2,6-Difluorphenyl)-4-(2,4,6-trichlorphenyl)-4H-5,6-dihydro-1,3,4-oxadiazin vom Schmelzpunkt 107-108°C.

### Beispiel 2

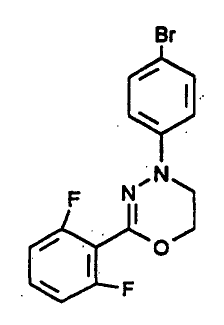

### (Verfahren a)

Zu einem Gemisch von 9,81 g (30,0 mmol) 1-(2,6-Difluorbenzoyl)-2-(4-bromphenyl)-hydrazin und 3,81 g (30,0 mmol) 1-Brom-2-fluorethan in 70 ml absolutem Ethanol werden bei Raumtemperatur 2,50 g (62,5 mmol) Natriumhydroxid in 8 ml Wasser getropft. Nach 5-stündigem Rühren erhitzt man noch weitere 5 Stunden auf 60°C.
Die abgekühlte Lösung wird zwischen Methyl-tert.-butylether und Wasser verteilt, die organische Phase abgetrennt und über Natriumsulfat getrocknet. Nach Säulenchromatographie (Kieselgel 60; Essigester/Hexan 1:3) erhält man 1,60 g (15% der Theorie) 4-(4-Bromphenyl)-2-(2,6-difluorphenyl)-4H-5,6-dihydro-1,3,4-oxadiazin vom Schmelzpunkt 138-140°C.

### Beispiel 3

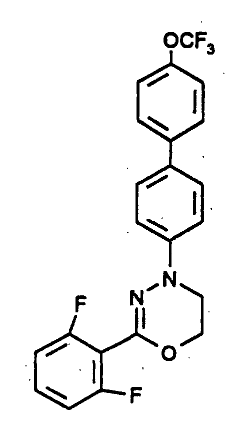

### (Verfahren e)

0,70 g (2,0 mmol) 4-(4-Bromphenyl)-2-(2,6-difluorphenyl)-4H-5,6-dihydro-1,3,4-oxadiazin (Bsp. 2) und 0,42 g (2,0 mmol) wasserfeuchte 4-Trifluormethyoxyphenylboronsäure werden mit 2,76 g (20 mmol) gemahlenem Kaliumcarbonat in 6 ml Toluol/Wasser/Ethanol (2:1:1) über Nacht bei 100°C gerührt.
Nach Abkühlen wird die Reaktionsmischung mit Essigester verdünnt und die organische Phase abgetrennt. Nach Trocknen über Natriumsulfat wird im Vakuum eingeengt. Der Rückstand wird in heißem Essigester aufgenommen und das Produkt mit Hexan ausgefällt.
Man erhält 0,8 g (93% der Theorie) 4-[4-(4-Trifluormethoxyphenyl)phenyl]-2-(2,6-difluorphenyl)-4H-5,6-dihydro-1,3,4-oxadiazin vom Schmelzpunkt 130-133°C.

### Beispiel 4

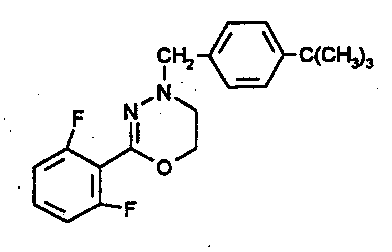

### (Verfahren a)

Zu einer Lösung von 12,0 g (37,7 mmol) 2-(4-tert.-Butylbenzyl)-2,6-difluorbenzoesäurehydrazid in 50 ml Ethanol gibt man bei Raumtemperatur 5,0 g (39,4 mmol) 1,2-Bromfluorethan. Zu diesem Gemisch tropft man eine Lösung von 3,2 g (80 mmol) Natriumhydroxid in 20 ml Wasser und erhitzt 18 Stunden unter Rückfluß. Absaugen und Trocknen des entstandenen weißen Niederschlags liefert 4,8 g (37% der Theorie) 2-(2,6-Difluorphenyl)-4-(4-tert.-butylbenzyl)-4H-5,6-dihydro-1,3,4-oxadiazin vom Schmelzpunkt 62-64°C.

### Herstellung des Ausgangsproduktes

### Beispiel (II-1)

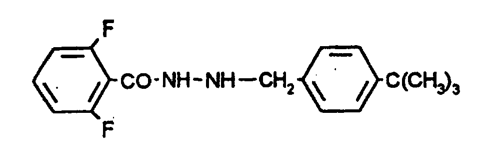

15,8 g (50 mmol) 2-(4-tert.-Butylphenyl)methylen-2,6-difluorbenzoesäurehydrazid werden in 120 ml Ethanol gelöst und das Gemisch nach Zugabe von 1 g 10%-iger Palladium/Kohle 8 Stunden bei 3 bar und Raumtemperatur hydriert. Das nach Abfiltrieren des Katalysators und Entfernen des Lösungsmittels erhaltene blaßgelbe Öl wird an Kieselgel (Ethylacetat/Hexan 1:4) chromatographiert. Man erhält 11,4 g (72% der Theorie) 2-(4-tert.-Butylbenzyl)-2,6-difluorbenzoesäurehydrazid als farbloses Öl.

### Beispiel (XI-1)

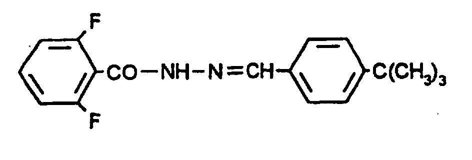

9,0 g (53 mmol) 2,6-Difluorbenzoesäurehydrazid und 8,6 g (53 mmol) 4-tert.-Butylbenzaldehyd werden in 150 ml Toluol gelöst und 16 Stunden unter Rückfluß am Wasserabscheider erhitzt. Entfernen des Lösungsmittels unter vermindertem Druck liefert 16,5 g (98% der Theorie) 2-(4-tert.-Butylphenyl)methylen-2,6-difluorbenzoesäurehydrazid als gelbliches Öl, das direkt weiter umgesetzt wird.

Analog zu den Herstellungsbeispielen 1 bis 4 bzw. gemäß den allgemeinen Angaben zur Herstellung erhält man die folgenden Verbindungen der Formel (I)

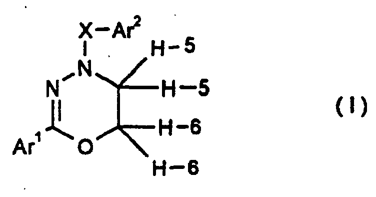

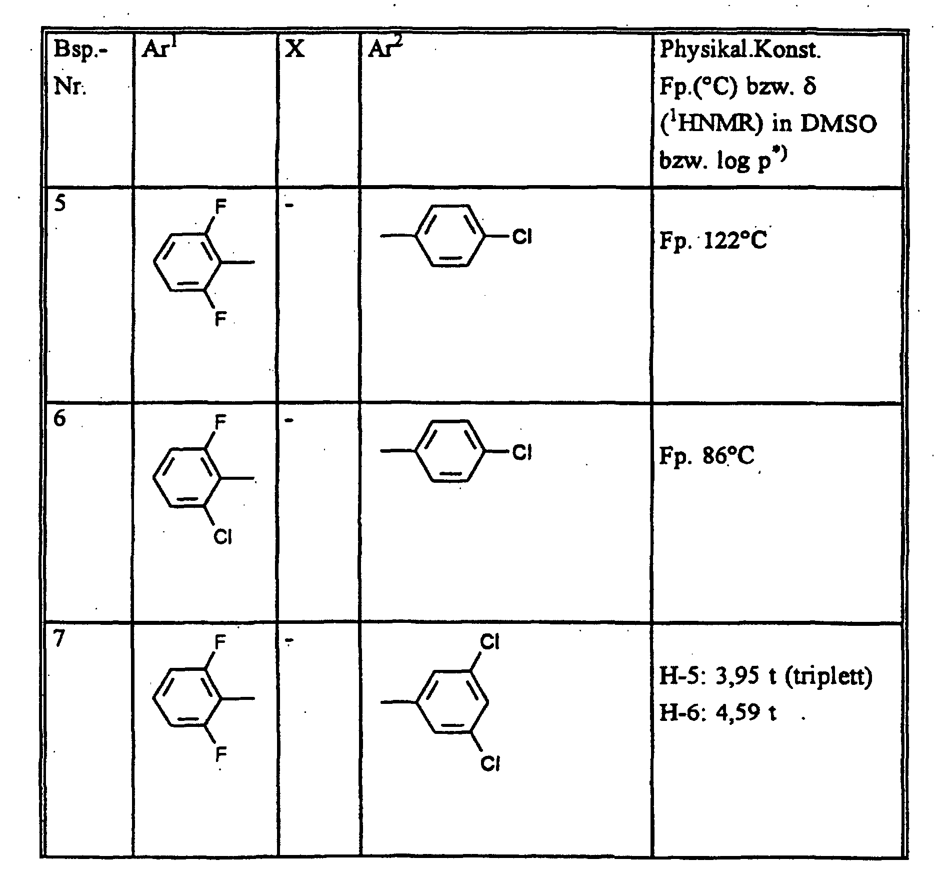

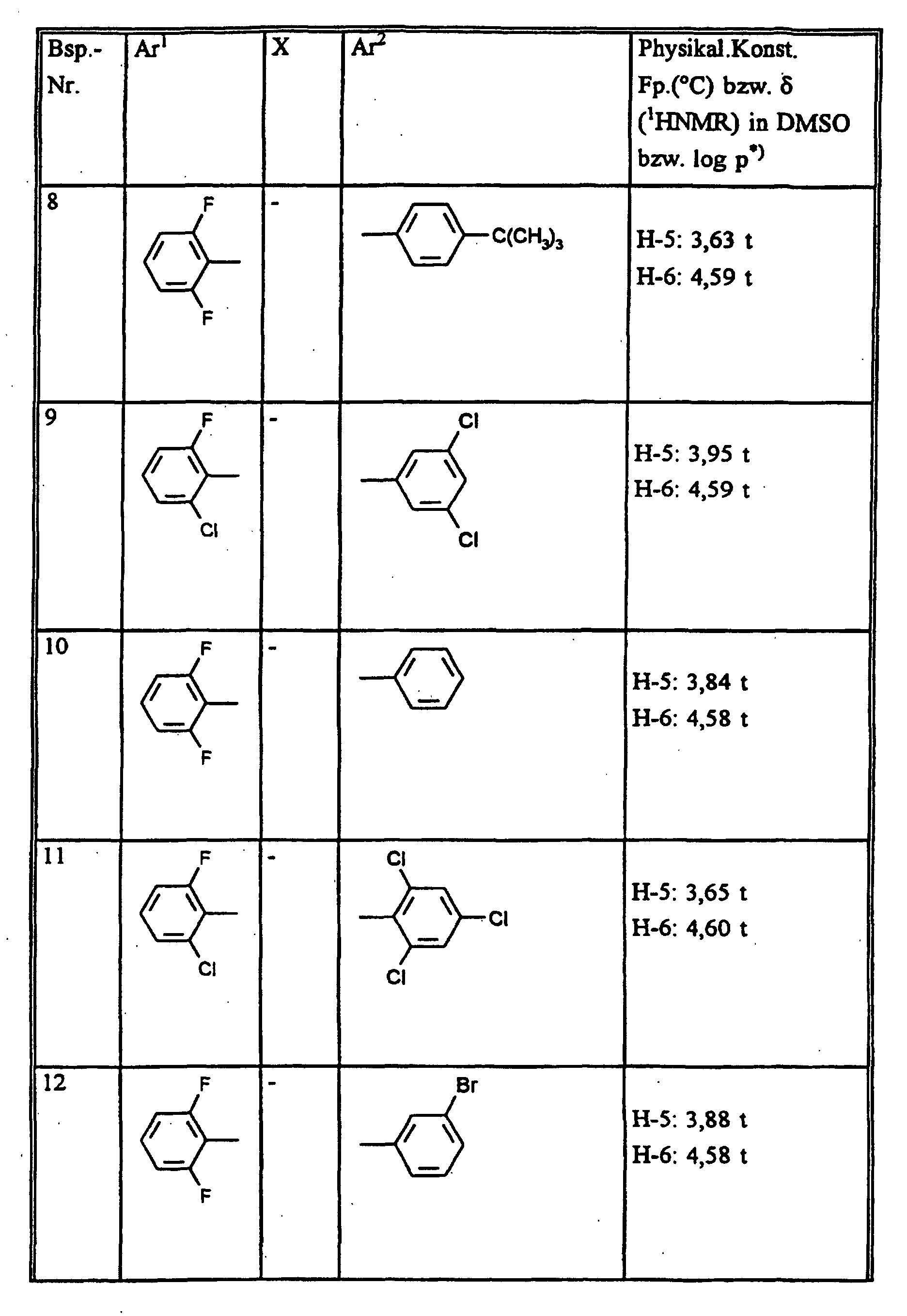

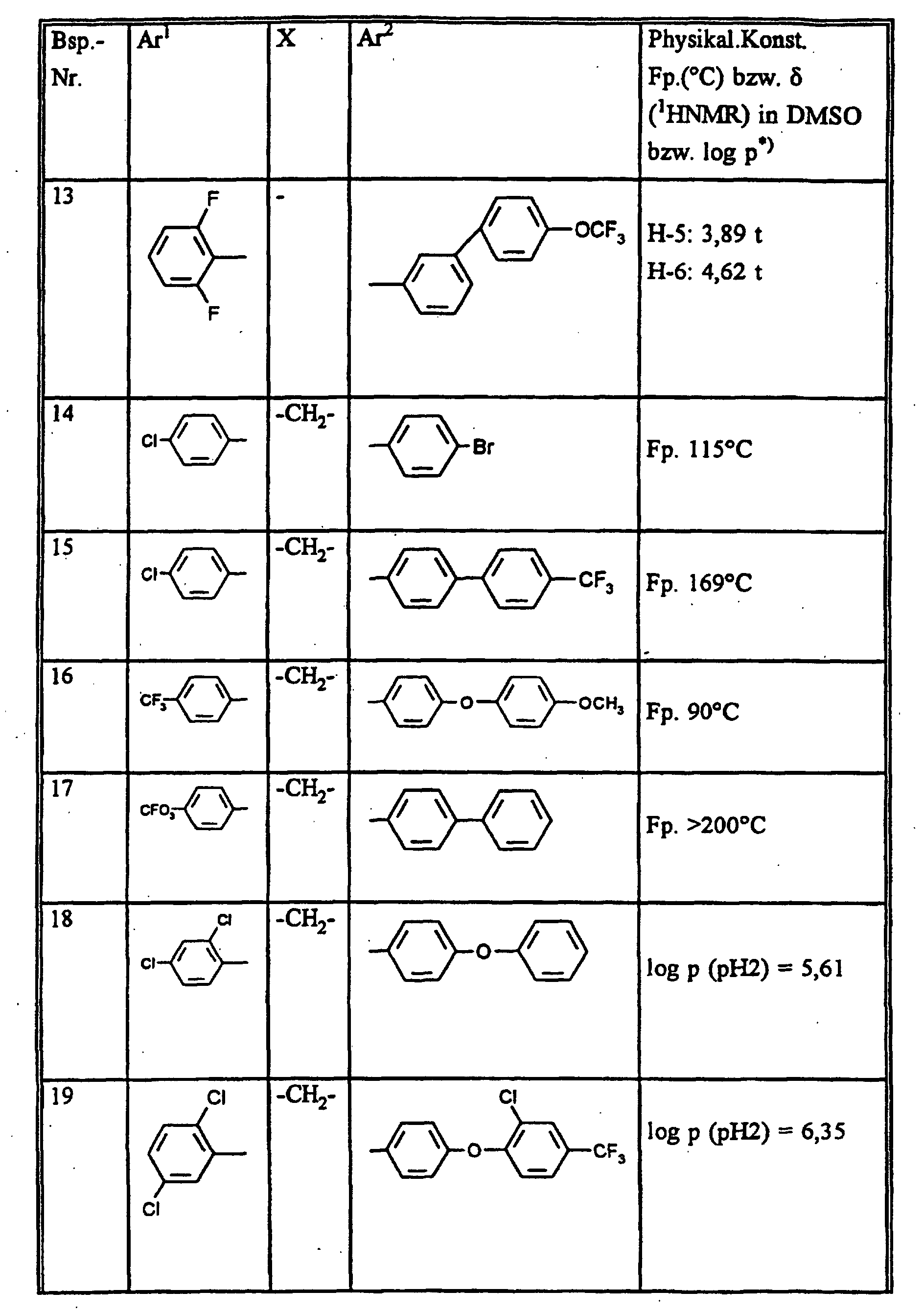

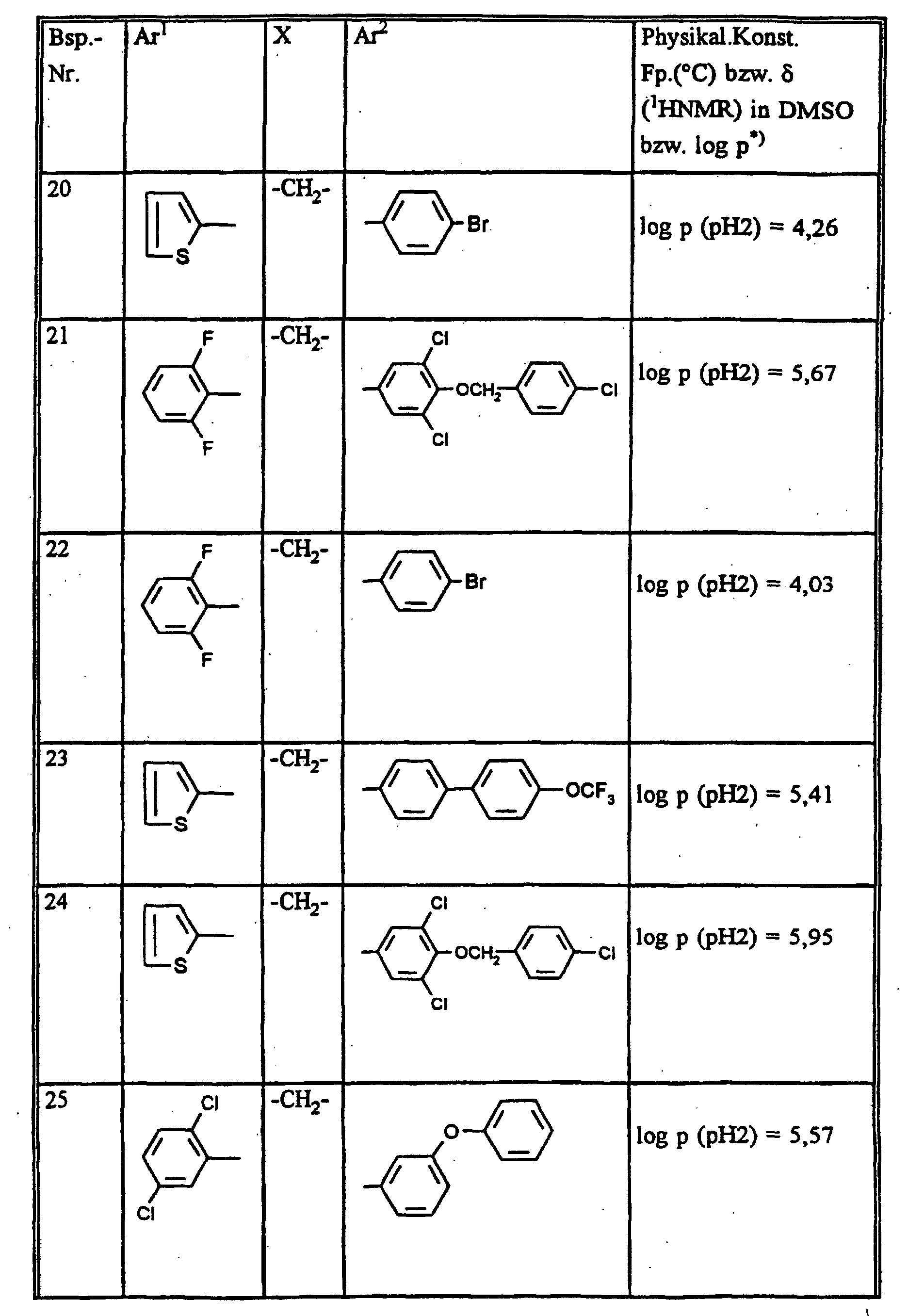

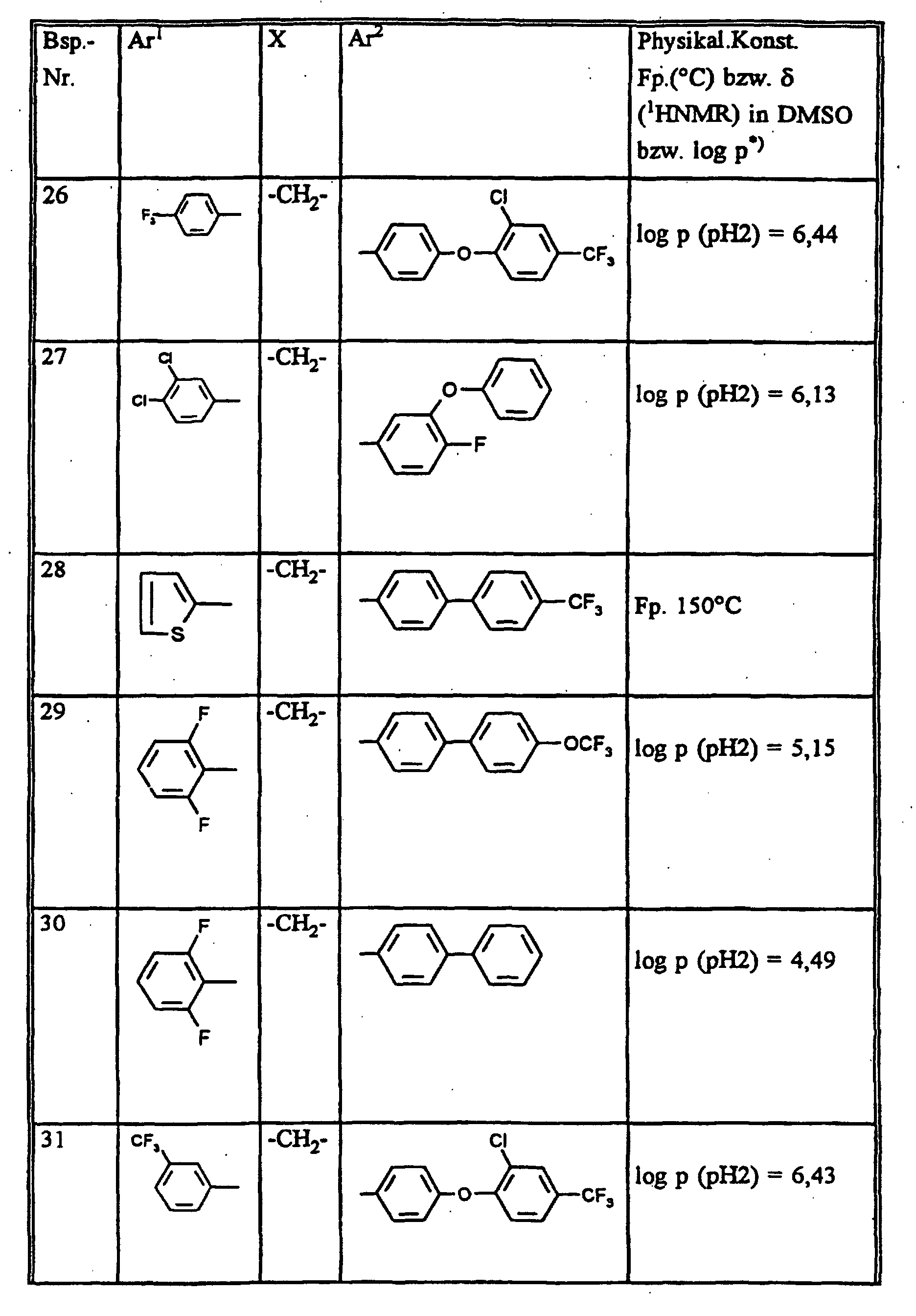

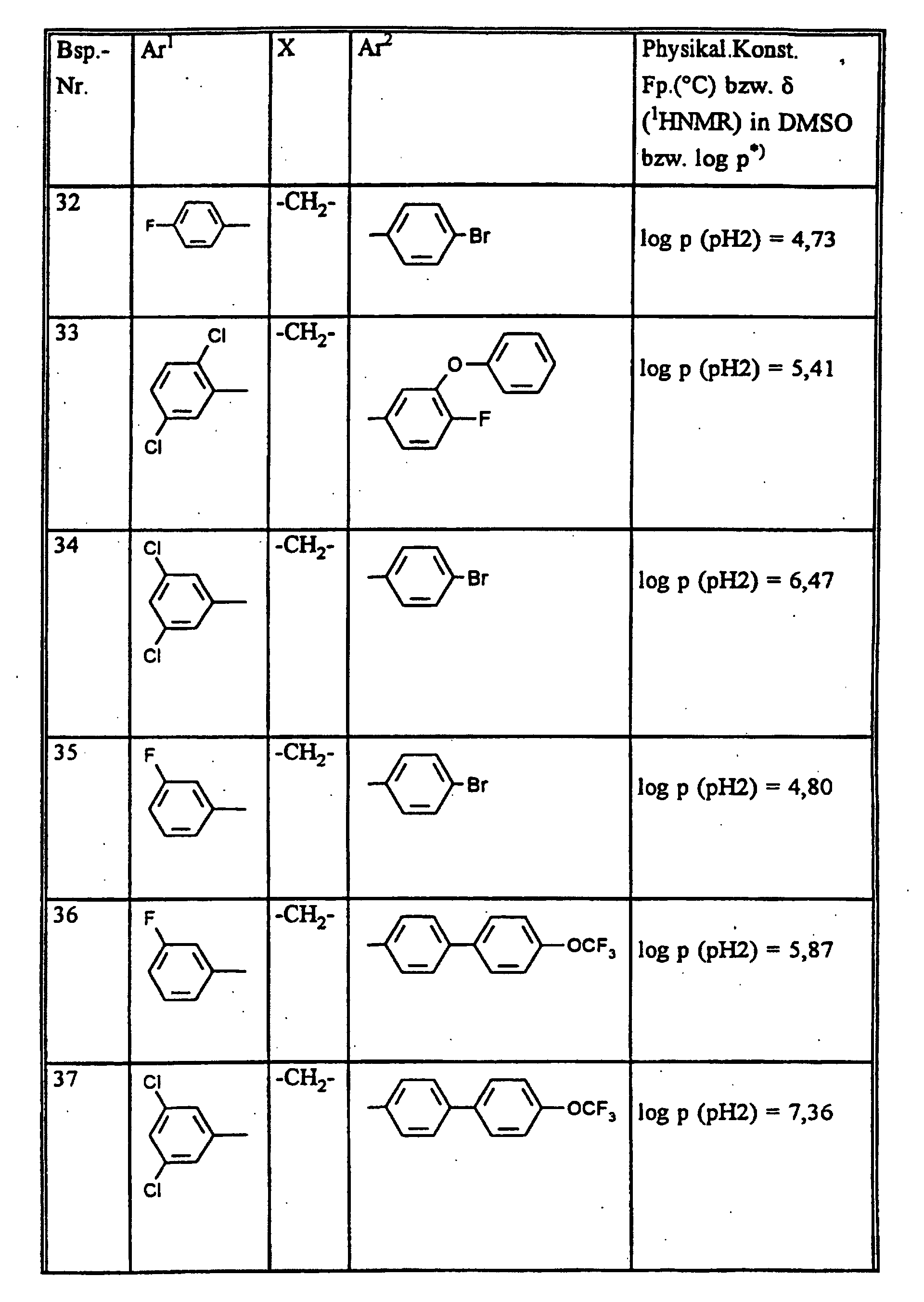

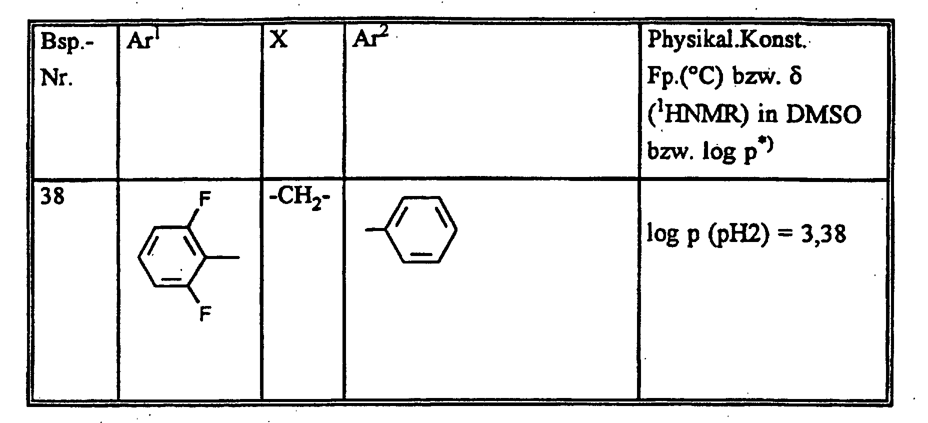

### Anwendungsbeispiele

In dem nachfolgenden Anwendungsbeispiel wird die aus Pesticide Biochemistry ahd Physiology 47, 1-7 (1993) bekannte Verbindung (A) als Vergleichssubstanz eingesetzt: 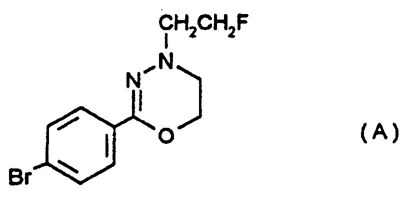 2-(4-Bromphenyl)-4-(2-fluoreth-1-yl)-4H-5,6-dihydro-1,3,4-oxadiazin

### Beispiel A

### Tetranychus-Test (OP-resistent/Spritzbehandlung)

| | |
|---|---|
| Lösungsmittel | 3 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus wlgaris), die stark von allen Stadien der gemeinen Spinnmilbe (*Tetranychus urticae*) befallen sind, werden mit einer Wirkstoffzubereitung der gewünschten Konzentration gespritzt.

Nach der gewünschten Zeit wird die Wirkung in % bestimmt. Dabei bedeutet 100%, daß alle Spinnmilben abgetötet wurden; 0% bedeutet, daß keine Spinnmilben abgetötet wurden.

In diesem Test bewirkten z.B. die Verbindungen gemäß den Herstellungsbeispielen 3 bzw. 4 bei einer beispielhaften Wirkstoffkonzentration von 0,001% nach 5 Tagen eine Abtötung von 95% bzw. 98%, während die bekannte Verbindung (A) keine Wirkung zeigte.

## Patentansprüche

1. Verbindungen der Formel (I) 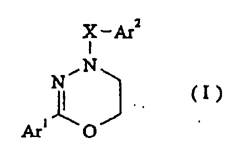 in welcher
Ar¹ für Phenyl steht, das gegebenenfalls einfach bis dreifach, gleich oder verschieden substituiert ist durch Fluor, Chlor, Brom, C₁-C₄-Alkyl, C₁-C₄-Alkoxy, C₁-C₄-Alkylthio, einfach bis fünffach, gleich oder verschieden durch Fluor oder Chlor substituiertes C₁-C₂-Alkyl, einfach bis fünffach, gleich oder verschieden durch Fluor oder Chlor substituiertes C₁-C₄-Alkoxy, SCF₃, SCHF₂, Nitro oder Cyano oder für Thienyl,
Ar² für Phenyl steht, das gegebenenfalls einfach bis vierfach, gleich oder verschieden substituiert ist durch
Fluor, Chlor, Brom, C₁-C₁₈-Alkyl, C₁-C₆-Alkoxy-C₁-C₈-alkyl,
einfach bis sechsfach, gleich oder verschieden durch Fluor oder Chlor substituiertes C₁-C₈-Alkoxy,
einfach bis fünffach, gleich oder verschieden durch Fluor oder Chlor substituiertes C₁-C₂-Alkyl,
C₁-C₁₈-Alkoxy, -(OC₂H₄)₁₋₃-O-C₁-C₆-alkyl, C₁-C₁₂-Alkylthio,
einfach bis sechsfach, gleich oder verschieden durch Fluor oder Chlor substituiertes C₁-C₈-Alkylthio,
jeweils gegebenenfalls durch C₁-C₄-Alkyl, C₁-C₄-Alkoxy, Cyclohexyl oder Phenyl substituiertes Cyclohexyl oder Cyclohexyloxy, gegebenenfalls einfach oder zweifach, gleich oder verschieden durch Fluor, Chlor oder CF₃ substituiertes Pyridyloxy,
jeweils gegebenenfalls einfach bis dreifach, gleich oder verschieden durch
C₁-C₁₂-Alkyl, Fluor, Chlor, Brom, Cyano, CF₃, C₁-C₄-Alkoxy, einfach bis sechsfach, gleich oder verschieden durch Fluor oder Chlor substituiertes C₁-C₄-Alkoxy, C₁-C₄-Alkoxy-C₁-C₄-alkyl, C₁-C₄-Alkoxyethylenoxy, C₁-C₄-Alkylthio, einfach bis sechsfach, gleich oder verschieden durch Fluor oder Chlor substituiertes C₁-C₄-Alkylthio oder Trimethylsilyl substituiertes Phenyl, Benzyl, Phenethyl, Phenoxy, Phenoxymethyl, Phenylthio, Phenethyloxy, Benzyloxy, Benzylthio oder Styryl,
X für die Gruppierung -(CHR¹)ₘ-(CHR²)ₙ- steht, worin
R¹ für Wasserstoff steht,
R² für Wasserstoff steht,
m für 0 oder 1 steht und
n für 0 oder 1 steht.

2. Verfahren zur Herstellung von Verbindungen der Formel (I) gemäß Anspruch 1, **dadurch gekennzeichnet, daß** man
a) Säurehydrazide der Formel (II)
Ar¹-CO-NH-NH-X-Ar² (II)
in welcher
Ar¹, Ar² und X die in Anspruch 1 angegebene Bedeutung haben,
mit Ethan-Derivaten der Formel (III)
Y¹-CH₂-CH₂-Y² (III)
in welcher
Y¹ und Y² gleich oder verschieden sind und jeweils für eine Abgangsgruppe stehen,
in Gegenwart einer Base und in Gegenwart eines Verdünnungsmittels umsetzt; oder
b) Säure,hydrazide der Formel (IV) 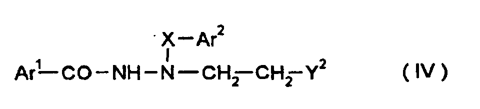 in welcher
Ar¹, Ar², X und Y² die oben angegebene Bedeutung haben,
gegebenenfalls in Gegenwart eines Katalysators, gegebenenfalls in Gegenwart einer Base und gegebenenfalls in Gegenwart eines Verdünnungsmittels cyclisiert;
oder
c) Säurehydrazide der Formel (V) 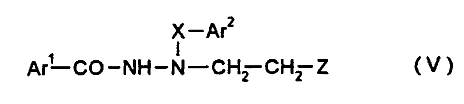 in welcher
Ar¹, Ar² und X die oben angegebene Bedeutung haben und
Z für -OH oder -O-COR steht, wobei
R für Alkyl steht,
in Gegenwart einer Säure und gegebenenfalls in Gegenwart eines Verdünnungsmittels cyclisiert;
oder
d) 1,3,4-Oxadiazin-Derivate der Formel (VI) 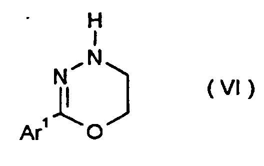 in welcher
Ar¹ die oben angegebene Bedeutung hat,
mit Verbindungen der Formel (VII)
Y¹-(CHR¹)ₘ-(CHR²)ₙ-Ar² (VII)
in welcher
Ar², R¹, R², Y¹, m und n die oben angegebene Bedeutung haben, wobei mindestens einer der Indizes m oder n für 1 steht,
in Gegenwart einer Base und gegebenenfalls in Gegenwart eines Verdünnungsmittels umsetzt;
oder
e) 1,3,4-Oxadiazin-Derivate der Formel (Ia) 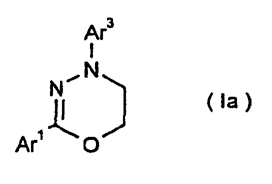 in welcher
Ar¹ die oben angegebene Bedeutung hat und
Ar³ für Halogenaryl oder Halogenhetaryl steht
mit Boronsäuren der Formel (VIII)
Ar⁴-B(OH)₂ (VIII)
in welcher
Ar⁴ für gegebenenfalls substituiertes Aryl steht
in Gegenwart einer Base und in Gegenwart eines Verdünnungsmittels umsetzt.

3. Verbindungen der Formel (I) gemäß Anspruch 1, in welcher
Ar¹ für Phenyl steht, das einfach bis dreifach, gleich oder verschieden substituiert ist durch Fluor, Chlor, Trifluormethyl oder Trifluormethoxy oder für Thienyl steht,
Ar² für Phenyl steht, das gegebenenfalls einfach bis dreifach, gleich oder verschieden substituiert ist durch Fluor, Chlor, Brom, C₁-C₆-Alkyl oder durch jeweils gegebenenfalls einfach oder zweifach, gleich oder verschieden durch Fluor, Chlor, Trifluormethyl oder Trifluormethoxy substituiertes Phenyl, Phenoxy oder Benzyloxy und
X für die Gruppierung -(CH₂)ₘ- steht, worin
m für 0 oder 1 steht

4. Schädlingsbekämpfungsmittel, **gekennzeichnet durch** einen Gehalt an mindestens einer Verbindung der Formel (I) gemäß Anspruch 1.

5. Verwendung von Verbindungen der Formel (I) gemäß Anspruch 1 zur Bekämpfung von Schädlingen.

6. Verfahren zur Bekämpfung von Schädlingen, **dadurch gekennzeichnet, daß** man von Verbindungen der Formel (I) gemäß Anspruch 1 auf Schädlinge und/oder ihren Lebensraum einwirken läßt.

7. Verfahren zur Herstellung von Schädlingsbekämpfungsmitteln, **dadurch gekennzeichnet, daß** man Verbindungen der Formel (I) gemäß Anspruch 1 mit Streckmitteln und/oder oberflächenaktiven Mitteln vermischt.

8. Verwendung von Verbindungen der Formel (I) gemäß Anspruch 1 zur Herstellung von Schädlingsbekämpfungsmitteln.

## Claims

1. Compounds of the formula (I) 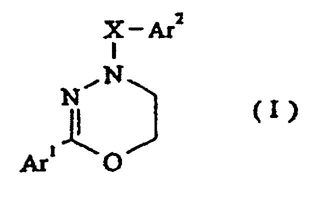 in which
Ar¹ represents phenyl which is optionally monosubstituted to trisubstituted by identical or different substituents from the series consisting of fluorine, chlorine, bromine, C₁-C₄-alkyl, C₁-C₄-alkoxy, C₁-C₄-alkylthio, C₁-C₂-alkyl which is monosubstituted to pentasubstituted by identical or different fluorine or chlorine substituents, C₁-C₄-alkoxy which is monosubstituted to pentasubstituted by identical or different fluorine or chlorine substituents, SCF₃, SCHF₂, nitro and cyano, or represents thienyl,
Ar² represents phenyl which is optionally monosubstituted to tetrasubstituted by identical or different substituents from the series consisting of
fluorine, chlorine, bromine, C₁-C₁₈-alkyl, C₁-C₆-alkoxy-C₁-C₈-alkyl,
C₁-C₈-alkoxy which is monosubstituted to hexasubstituted by identical or different fluorine or chlorine substituents,
C₁-C₂-alkyl is monosubstituted to pentasubstituted by identical or different fluorine or chlorine substituents,
C₁-C₁₈-alkoxy, -(OC₂H₄)₁₋₃-O-C₁-C₆-alkyl, C₁-C₁₂-alkylthio,
C₁-C₈-alkylthio which is monosubstituted to hexasubstituted by identical or different fluorine or chlorine substituents,
cyclohexyl or cyclohexyloxy, each of which is optionally substituted by C₁-C₄-alkyl, C₁-C₄-alkoxy, cyclohexyl or phenyl,
pyridyloxy which is optionally monosubstituted or disubstituted by identical or different substituents from the series consisting of fluorine, chlorine and CF₃,
and phenyl, benzyl, phenethyl, phenoxy, phenoxymethyl, phenylthio, phenethyloxy, benzyloxy, benzylthio or styryl, each of which is optionally monosubstituted to trisubstituted by identical or different substituents from the series consisting of C₁-C₁₂-alkyl, fluorine, chlorine, bromine, cyano, CF₃, C₁-C₄-alkoxy, C₁-C₄-alkoxy which is monosubstituted to hexasubstituted by identical or different fluorine or chlorine substituents, C₁-C₄-alkoxy-C₁-C₄-alkyl, C₁-C₄-alkoxyethylenoxy, C₁-C₄-alkylthio, C₁-C₄-alkylthio which is monosubstituted to hexasubstituted by identical or different fluorine or chlorine substituents, and trimethylsilyl,
X represents the group -(CHR¹)ₘ-(CHR²)ₙ-, in which
R¹ represents hydrogen,
R² represents hydrogen,
m represents 0 or 1 and
n represents 0 or 1.

2. Process for the preparation of compounds of the formula (I) according to Claim 1, **characterized in that**
a) acid hydrazides of formula (II)
Ar¹-CO-NH-NH-X-Ar² (II)
in which
Ar¹, Ar² and X have the meanings given in Claim 1,
are reacted with ethane derivatives of the formula (III)
Y¹-CH₂-CH₂-Y² (III)
in which
Y¹ and Y² are identical or different and represent in each case a leaving group
in the presence of a base and in the presence of a diluent;
or
b) acid hydrazides of the formula (IV) 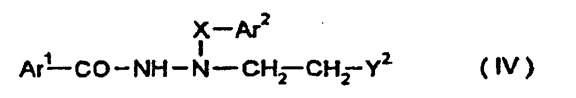 in which
Ar¹, Ar², X and Y² have the abovementioned meanings,
are cyclized, if appropriate in the presence of a catalyst, if appropriate in the presence of a base and if appropriate in the presence of a diluent;
or
c) acid hydrazides of the formula (V) 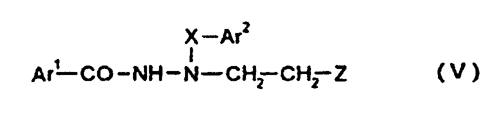 in which
Ar¹, Ar² and X have the abovementioned meanings and
Z represents -OH or -O-COR, where
R represents alkyl
are cyclized in the presence of an acid and if appropriate in the presence of a diluent;
or
d) 1,3,4-oxadiazine derivatives of the formula (VI) 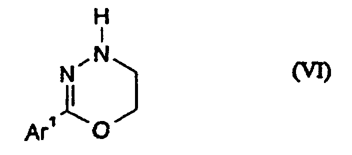 in which
Ar¹ has the abovementioned meanings
are reacted with compounds of the formula (VII)
Y¹-(CHR¹)ₘ-(CHR²)ₙ-Ar² (VII)
in which
Ar², R¹, R², Y¹, m and n have the abovementioned meanings, at least
one of the indices m and n representing 1
in the presence of a base and if appropriate in the presence of a diluent;
or
e) 1,3,4-oxadiazine derivatives of the formula (Ia) 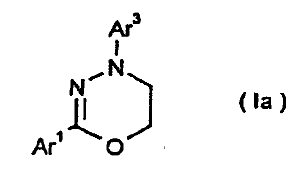 in which
Ar¹ has the abovementioned meanings and
Ar³ represents halogenoaryl or halogenohetaryl
are reacted with boronic acids of the formula (VIII)
Ar⁴-B(OH)₂ (VIII)
in which
Ar⁴ represents optionally substituted aryl
in the presence of a base and in the presence of a diluent.

3. Compounds of the formula (I) according to Claim 1 in which
Ar¹ represents phenyl which is monosubstituted to trisubstituted by identical or different substituents from the series consisting of fluorine, chlorine, trifluoromethyl and trifluoromethoxy, or represents thienyl.
Ar² represents phenyl which is optionally monosubstituted to trisubstituted by identical or different substituents from the series consisting of fluorine, chlorine, bromine, C₁-C₆-alkyl, or by phenyl, phenoxy or benzyloxy, each of which is optionally monosubstituted or disubstituted by identical or different substituents from the series consisting of fluorine, chlorine, trifluoromethyl and trifluoromethoxy.
X represents the group -(CH₂)ₘ-, in which
m is 0 or 1.

4. Pesticides, **characterized in that** they comprise at least one compound of the formula (I) according to Claim 1.

5. Use of compounds of the formula (I) according to Claim 1 for combating pests.

6. Method of combating pests, **characterized in that** compounds of the formula (I) according to Claim 1 are allowed to act on pests and/or their environment.

7. Process for the preparation of pesticides, **characterized in that** compounds of the formula (I) according to Claim 1 are mixed with extenders and/or surface-active agents.

8. Use of compounds of the formula (I) according to Claim 1 for the preparation of pesticides.

## Revendications

1. Composés de formule (I) 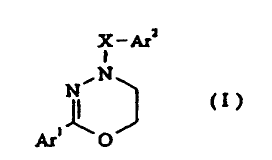 dans laquelle
Ar¹ représente un reste phényle qui est éventuellement substitué une à trois fois identiques ou différentes par du fluor, du chlore, du brome, un radical alkyle en C₁ à C₄, alkoxy en C₁ à C₄, alkylthio en C₁ à C₄, alkyle en C₁ ou C₂ substitué une à cinq fois identiques ou différentes par du fluor ou du chlore, alkoxy en C₁ à C₄ substitué une à cinq fois identiques ou différentes par du fluor ou du chlore, SCF₃, SCHF₂, nitro ou cyano,
ou un reste thiényle,
Ar² est un reste phényle qui est éventuellement substitué une à quatre fois identiques ou différentes par
du fluor, du chlore, du brome, un radical alkyle en C₁ à C₁₈, (alkoxy en C₁ à C₆) (alkyle en C₁ à C₈),
alkoxy en C₁ à C₈ substitué une à six fois identiques ou différentes par du fluor ou du chlore,
alkyle en C₁ ou C₂ substitué une à cinq fois identiques ou différentes par du fluor ou du chlore,
alkoxy en C₁ à C₁₈, -(OC₂H₄)₁₋₃-O(alkyle en C₁. à C₆), alkylthio en C₁ à C₁₂, alkylthio en C₁ à C₈ substitué une à six fois identiques ou différentes par du fluor ou du chlore,
cyclohexyle ou cyclohexyloxy substitué chacun le cas échéant par un radical alkyle en C₁ à C₄, alkoxy en C₁ à C₄, cyclohexyle ou phényle,
pyridyloxy éventuellement substitué une ou deux fois identiques ou différentes par du fluor, du chlore ou un radical CF₃,
phényle, benzyle, phénéthyle, phénoxy, phénoxyméthyle, phénylthio, phénéthyloxy, benzyloxy, benzylthio ou styryle dont chacun est éventuellement substitué une à trois fois, identiques ou différentes, par
un radical alkyle en C₁ à C₁₂, du fluor, du chlore, du brome, un radical cyano, CF₃, alkoxy en C₁ à C₄, substitué une à six fois, identiques
ou différentes, par du fluor ou du chlore, (alkoxy en C₁ à C₄) (alkyle en C₁ à C₄), (alkoxy en C₁ à C₄)éthylénoxy, alkylthio en C₁ à C₄, alkylthio en C₁ à C₄ substitué une à six fois identiques ou différentes par du fluor ou du chlore, ou triméthylsilyle,
X représente le groupement -(CHR¹)ₘ-(CHR²)ₙ-, dans lequel
R¹ représente l'hydrogène,
R² représente l'hydrogène,
m a la valeur 0 ou 1 et
n a la valeur 0 ou 1.

2. Procédé de production de composés de formule (I) suivant la revendication 1, **caractérisé en ce que** :
a) on fait réagir des hydrazides d'acides de formule (II)
Ar¹ à CO-NH-NH-X-Ar² (II)
dans laquelle
Ar¹, Ar² et X ont la définition indiquée dans la revendication 1,
avec des dérivés d'éthane de formule (III)
Y¹ à CH₂ à CH₂-Y² (III)
dans laquelle
Y¹ et Y² sont identiques ou différents et représentent chacun un groupe partant,
en présence d'une base et en présence d'un diluant ; ou bien
b) on cyclise des hydrazides d'acides de formule (IV) 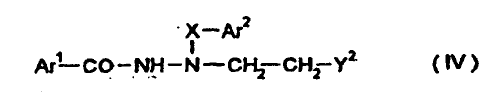 dans laquelle
Ar¹, Ar², X et Y² ont la définition indiquée ci-dessus,
éventuellement en présence d'un catalyseur, le cas échéant en présence d'une base et en présence éventuelle d'un diluant ;
ou bien
c) on cyclise des hydrazides d'acides de formule (V) 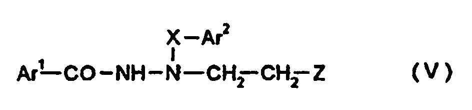 dans laquelle
Ar¹, Ar² et X ont la définition indiquée ci-dessus et
Z est un groupe -OH ou -O à COR, où
R est un radical alkyle,
en présence d'un acide et le cas échéant en présence d'un diluant ;
ou bien
d) on fait réagir des dérivés de 1,3,4-oxadiazine de formule (VI) 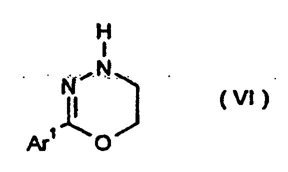 dans laquelle
Ar¹ a la définition indiquée ci-dessus,
avec des composés de formule (VII)
Y¹-(CHR¹)ₘ-(CHR²)ₙ-Ar² (VII)
dans laquelle
Ar², R¹, R², Y¹, m et n ont la définition indiquée ci-dessus,
l'un au moins des indices m ou n étant égal à 1, en présence d'une base et, le cas échéant, en présence d'un diluant ;
ou bien
e) on fait réagir des dérivés de 1,3,4-oxadiazine de formule (Ia) 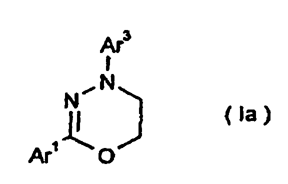 dans laquelle
Ar¹ a la définition indiquée ci-dessus et
Ar³ est un reste halogénaryle ou halogénhétaryle,
avec des acides boroniques de formule (VIII)
Ar⁴-B(OH)₂ (VIII)
dans laquelle
Ar⁴ est un reste aryle éventuellement substitué
en présence d'une base et en présence d'un diluant.

3. Composés de formule (I) suivant la revendication 1, dans laquelle
Ar¹ est un reste phényle qui est substitué une à trois fois identiques ou différentes par du fluor, du chlore, un radical trifluorométhyle ou trifluorométhoxy, ou un reste thiényle,
Ar² est un reste phényle qui est éventuellement substitué une à trois fois identiques ou différentes par du fluor, du chlore, du brome, un radical alkyle en C₁ à C₆ ou par un reste phényle, phénoxy ou benzyloxy dont chacun est éventuellement substitué une ou deux fois identiques ou différentes par du fluor, du chlore, un radical trifluorométhyle ou trifluorométhoxy, et
X représente le groupement -(CH₂)ₘ-, dans lequel
m a la valeur 0 ou 1

4. Compositions pesticides, **caractérisées par** une teneur en au moins un composé de formule (I) suivant la revendication 1.

5. Utilisation de composés de formule (I) suivant la revendication 1 pour combattre des parasites.

6. Procédé de lutte contre des parasites, **caractérisé en ce qu'**on fait agir des composés de formule (I) suivant la revendication 1 sur les parasites et/ou sur leur milieu.

7. Procédé de préparation de compositions pesticides, **caractérisé en ce qu'**on mélange des composés de formule (I) suivant la revendication 1 avec des diluants et/ou des agents tensioactifs.

8. Utilisation de composés de formule (I) suivant la revendication 1 pour la préparation de compositions pesticides.
